(19) Europäisches Patentamt / European Patent Office / Office européen des brevets

(11) **EP 1 881 847 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**07.12.2022 Patentblatt 2022/49**

(45) Hinweis auf die Patenterteilung:
**07.09.2016 Patentblatt 2016/36**

(21) Anmeldenummer: **06742996.9**

(22) Anmeldetag: **19.05.2006**

(51) Internationale Patentklassifikation (IPC):
**A61K 47/02** (2006.01)    **A61K 47/12** (2006.01)
**A61K 9/00** (2006.01)    **A61K 31/7088** (2006.01)
**A61K 47/48** (2006.01)    **C12N 15/87** (2006.01)

(52) Gemeinsame Patentklassifikation (CPC):
**A61K 39/00; A61K 9/0019; A61K 31/7088;
A61K 31/7105; A61K 47/02; A61K 47/12;
A61K 48/0008; A61K 48/005; A61P 35/00;
A61P 37/02; A61P 37/08; A61P 43/00;**
A61K 2039/53

(86) Internationale Anmeldenummer:
**PCT/EP2006/004784**

(87) Internationale Veröffentlichungsnummer:
**WO 2006/122828 (23.11.2006 Gazette 2006/47)**

(54) **OPTIMIERTE INJEKTIONSFORMULIERUNG FÜR MRNA**

INJECTION SOLUTION FOR RNA

SOLUTION D'INJECTION D'ARN

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.05.2005 DE 102005023170**

(43) Veröffentlichungstag der Anmeldung:
**30.01.2008 Patentblatt 2008/05**

(60) Teilanmeldung:
**16001381.9 / 3 153 179
19176589.0 / 3 583 953**

(73) Patentinhaber: **CureVac AG
72076 Tübingen (DE)**

(72) Erfinder:
• **HOERR, Ingmar
70176 Stuttgart (DE)**
• **PASCOLO, Steve
8004 Zürich (CH)**

(74) Vertreter: **Graf von Stosch, Andreas et al
Graf von Stosch
Patentanwaltsgesellschaft mbH
Prinzregentenstraße 22
80538 München (DE)**

(56) Entgegenhaltungen:
**WO-A-2006/008154    WO-A2-2005/009346**

DE-A1-102004 035 227    US-A- 5 766 903
US-A1- 2002 132 788    US-A1- 2002 165 183
US-A1- 2003 143 204    US-A1- 2004 106 567
US-A1- 2005 059 624    US-B2- 6 379 966

• CARRALOT J-P ET AL: "Polarization of immunity induced by direct injection of naked sequence-stabilized mRNA vaccines", CMLS CELLULAR AND MOLECULAR LIFE SCIENCES, BIRKHAUSER VERLAG, HEIDELBERG, DE, vol. 61, no. 18, 1 September 2004 (2004-09-01), pages 2418-2424, XP002355208, ISSN: 1420-682X, DOI: 10.1007/S00018-004-4255-0
• PASCOLO S: "Messenger RNA-based vaccines", EXPERT OPINION ON BIOLOGICAL THERAPY, INFORMA HEALTHCARE, ASHLEY, LONDON; GB, vol. 4, no. 8, 1 August 2004 (2004-08-01), pages 1285-1294, XP009095328, ISSN: 1471-2598, DOI: 10.1517/14712598.4.8.1285
• Bruce Alberts ET AL: "Control of gene expression" In: "Molecular Biology of the Cell", 1 January 2002 (2002-01-01), Garland Science, US, XP055222887, ISBN: 978-0-8153-4072-0 pages 448-449, * the whole document *
• Rausch et al; Human Vaccines & Immunotherapies 10:11;3146-3152: November 2014

EP 1 881 847 B2

**Beschreibung**

Die Erfindung betrifft die Verwendung von mRNA und einem wässrigen Injektionspuffer, der ein Natriumsalz, ein Calciumsalz, ein Kaliumsalz sowie ggf. Laktat enthält, zur Herstellung eines Arzneimittels, wobei eine mRNA Injektionslösung zur Steigerung des mRNA-Transfers und/oder der mRNA-Translation in einen/einem Wirtsorganismus bereitgestellt wird.

In der Therapie und Prävention zahlreicher Erkrankungen spielen molekularmedizinische Verfahren, wie die Gentherapie und die genetische Vakzinierung, eine große Rolle. Basis dieser Verfahren ist die Einbringung von Nukleinsäuren in Zellen bzw. Gewebe des Patienten, gefolgt von der Verarbeitung der durch die eingebrachten Nukleinsäuren kodierten Informationen, d.h. der Translation in die erwünschten Polypeptide bzw. Proteine. Als einzubringende Nukleinsäuren kommt hierbei sowohl DNA als auch RNA in Betracht.

Genetische Vakzinierungen, die aus der Injektion von nackter Plasmid-DNA bestehen, wurden erstmals in den frühen 90iger Jahren an Mäusen demonstriert. Es stellte sich jedoch in Studien der klinischen Phasen I/II heraus, dass diese Technologie beim Menschen nicht die durch die Studien an Mäusen geweckten Erwartungen erfüllen konnte[1]. Zahlreiche DNA-basierte genetische Vakzinierungen und Verfahren zur Einbringung von DNA in Zellen (u.a. Calciumphosphat-Transfektion, Polypren-Transfektion, Protoplasten-Fusion, Elektroporation, Mikroinjektion, Lipofektion, Verwendung von DNA-Viren als DNA-Vehikel) wurden seitdem entwickelt.

Vor 15 Jahren zeigten Wolff et al., dass die Injektion von nackter genetischer Information in Form von Plasmid-DNA (pDNA) oder mRNA bei Mäusen zu einer Proteinexpression führen kann[2]. Diesen Ergebnissen folgten Untersuchungen, die zeigten, dass nackte pDNA für eine Vakzinierung verwendet werden kann[3-5]. Die Verwendung von mRNA zur Vakzinierung fand jedoch bis in die späten 90iger weniger Beachtung, bis nachgewiesen wurde, dass der Transfer von mRNA in dendritischen Zellen Immunantworten auslöst[6]. Die direkte Injektion von nackter mRNA zur Vakzinierung blieb jedoch ein Randthema und wurde lediglich in vier Artikeln von drei unterschiedlichen Arbeitsgruppen behandelt[7-10]. Einer der Hauptgründe hierfür war die Instabilität von mRNA aufgrund ihrer raschen Zerstörung durch Ribonukleasen und der damit verbundenen eingeschränkten Effektivität der mRNA als genetisches Werkzeug *in vivo*. Zwischenzeitlich sind im Stand der Technik jedoch zahlreiche Verfahren zur Stabilisierung von mRNA beschrieben worden, so beispielsweise in EP-A-1083232, WO 99/14346, US 5,580,859 und US 6,214,804.

RNA als Nukleinsäure für ein genetisches Vehikel besitzt gegenüber DNA zahlreiche Vorteile, unter anderem:

i) die in die Zelle eingebrachte RNA integriert nicht in das Genom (während DNA in gewissem Ausmaß in das Genom integriert und dabei in ein intaktes Gen des Genoms der Wirtszelle insertieren kann, so dass dieses Gen mutieren kann und zu einem teilweisen oder vollständigen Verlust der genetischen Information oder zu einer Fehlinformation führen kann),

ii) es sind keine viralen Sequenzen, wie Promotoren etc., zur wirksamen Transkription von RNA erforderlich (während für die Expression von in die Zelle eingebrachter DNA ein starker Promotor (z.B., der virale CMV-Promotor) erforderlich ist. Die Integration derartiger Promotoren in das Genom der Wirtszelle kann zu unerwünschten Veränderungen der Regulierung der Genexpression führen),

iii) der Abbau eingebrachter RNA erfolgt in begrenzter Zeit (einige Stunden)[11,12] so dass eine transiente Genexpression erzielt werden kann, die nach der erforderlichen Behandlungsdauer eingestellt werden kann (während das bei in das Genom integrierter DNA nicht möglich ist).

iv) RNA führt nicht zu einer Induktion pathogener anti-RNA-Antikörper in dem Patienten (während die Induktion anti-DNA-Antikörper für die Hervorrufung einer unerwünschten Immunantwort bekannt ist),

v) RNA ist vielseitig einsetzbar - jede beliebige RNA für jedes beliebige Protein von Interesse kann kurzfristig für eine Vakzinierung hergestellt werden, sogar auf individueller Basis eines Patienten.

Zusammenfassend bleibt festzustellen, dass mRNA eine transiente Kopie der kodierten genetischen Information in allen Organismen darstellt, als Vorlage für die Synthese von Proteinen dient und im Gegensatz zu DNA alle erforderlichen Voraussetzungen darstellt, um einen geeigneten Vektor für den Transfer exogener genetischer Informationen *in vivo* bereitzustellen.

Eine besonders geeignete Vorgehensweise für den beschriebenen Transfer von Nukleinsäuren in einen Wirtsorganismus, insbesondere ein Säugetier, stellt deren Injektion dar. Während für solche Injektionen DNA üblicherweise in Wasser, NaCl oder Injektionspuffer PBS verdünnt wird, wird RNA üblicherweise nur in einem Injektionspuffer verdünnt. Als RNA-Injektionspuffer werden Standardpuffer, wie Phosphat-gepufferte Salzlösungen, insbesondere PBS und HEPES-gepufferte Salzlösung (HBS), verwendet. Bei dem Transfer von mRNA wird eine solche RNA-Injektionslösung vor ihrer Applikation vorzugsweise für kurze Zeit erhitzt, um Sekundärstrukturen der mRNA zu entfernen. Ein Nachteil bei der Verwendung dieser Standardpuffer für die RNA-Injektionslösungen ist, dass der intradermale Transfer der RNA nur sehr ineffizient erfolgt. Ein weiterer Nachteil ist, dass die Translationsrate der transferierten RNA sehr gering ist. Ein weiterer Nachteil ist darin begründet, dass die RNA in solchen Standardpuffern häufig eine Sekundärstruktur (z.B. eine sog. hair pin (Haarnadel) Struktur) ausbildet, welche die Effektivität der Aufnahme der RNA in das Cytosol stark herabsetzen kann.

So untersuchten Carralot et al. Globin-stabilisierte mRNA-basierte Vakzinierung in Mäusen.[13] Dabei wurde nackte β-globin-UTR-stabilisierte mRNA, die für β-Galaktosidase codiert, in HEPES-gepuffertem Injektionspuffer (150 mM NaCl, 10 mM HEPES) Mäusen injiziert und eine in vivo Translation detektiert. Eine solche Injektion löste eine antigen-spezifische T-Helfer 2 (Th2) Immunantwort in den Mäusen aus.

WO 2005/009346 A2, US 2003/143204 A1, US 2007/132788 A1 und US 2009/106567 A1 offenbaren die Verabreichung einer siRNA in einer Ringer-Lösung.

Die WO 2006/008154 offenbart ein Gemisch, das mRNA zur Vakzinierung enthält, wobei mindestens eine mRNA einen für mindestens ein Antigen aus einem Tumor codierenden Bereich enthält und mindestens eine weitere mRNA einen für mindestens ein immunogenes Protein (Polypeptid) codierenden Bereich enthält. Dabei wurde ein entsprechendes mRNA-Antigen-Gemisch in einer Ringer-Laktat-Lösung Patienten verabreicht. Zudem wurde mRNA, die für Luziferase aus Photinus pyralis codiert, in einer Ringer-Laktat-Lösung Mäusen injiziert.

Aufgabe der vorliegenden Erfindung ist es daher, ein System bereitzustellen, mit dem zum einen der mRNA-Transfer in einen Wirtsorganismus verbessert wird und zum anderen die Translationsrate der übertragenen mRNA erhöht wird.

Diese Aufgabe wird durch die in den Ansprüchen gekennzeichneten Ausführungsformen der Erfindung gelöst.

Ein Gegenstand der vorliegenden Erfindung ist die Verwendung von mRNA und einem wässrigen Injektionspuffer, der ein Natriumsalz, bevorzugt mindestens 50 mM eines Natriumsalzes, ein Calciumsalz, bevorzugt mindestens 0,01 mM eines Calciumsalzes, und ein Kaliumsalz, bevorzugt mindestens 3 mM eines Kaliumsalzes, enthält, zur Herstellung eines Arzneimittels gemäß Anspruch 1. Eine mRNA-Injektionslösung zur Verwendung in der Behandlung gemäß Anspruch 11 ist Gegenstand eines weiteren Aspekts der vorliegenden Erfindung. Die mRNA-Injektionslösung ergibt sich also aus dem Injektionspuffer und der im Injektionspuffer gelösten mRNA.

Gemäß einer bevorzugten Ausführungsform liegen die im Injektionspuffer enthaltenen Natriumsalze, Calciumsalze und Kaliumsalze in Form von Halogeniden, z.B. Chloriden, Iodiden oder Bromiden, in Form ihrer Hydroxide, Carbonate, Hydrogencarbonate oder Sulfate vor. Beispielhaft sollen hier für das Natriumsalz $NaCl$, $NaI$, $NaBr$, $Na_2CO_3$, $NaHCO_3$, $Na_2SO_4$, für das enthaltene Kaliumsalz $KCl$, $KI$, $KBr$, $K_2CO_3$, $KHCO_3$, $K_2SO_4$, und für das Calciumsalz $CaCl_2$, $CaI_2$, $CaBr_2$, $CaCO_3$, $CaSO_4$, $Ca(OH)_2$ genannt werden. Auch können organische Anionen der vorgenannten Kationen im Injektionspuffer enthalten sein.

In einer besonders bevorzugten Ausführungsform der erfindungsgemäßen Verwendung von mRNA und einem Injektionspuffer enthält ein erfindungsgemäß eingesetzter Injektionspuffer als Salze Natriumchlorid ($NaCl$), Calciumchlorid ($CaCl_2$) und Kaliumchlorid ($KCl$), wobei neben den Chloriden auch andere Anionen enthalten sein können. Typischerweise liegen diese Salze im Injektionspuffer in einer Konzentration von mindestens 50 mM Natriumchlorid ($NaCl$), mindestens 3 mM Kaliumchlorid ($KCl$) und mindestens 0,01 mM Calciumchlorid ($CaCl_2$) vor.

Der eingesetzte Injektionspuffer kann sowohl als hypertonischer als auch isotonischer oder hypotonischer Injektionspuffer vorliegen. Im Zusammenhang mit der vorliegenden Erfindung ist dabei der Injektionspuffer jeweils in Bezug auf das jeweilige Referenzmedium hypertonisch, isotonisch oder hypotonisch, d.h. der erfindungsgemäß eingesetzte Injektionspuffer weist entweder einen im Vergleich mit dem jeweiligen Referenzmedium höheren, gleichen oder niedrigeren Salzgehalt auf, wobei bevorzugt solche Konzentrationen der zuvor genannten Salze eingesetzt werden, die nicht zu einer durch Osmose oder anderen Konzentrationseffekten bedingten Schädigung der Zellen führen. Referenzmedien sind hier beispielsweise "in vivo"-Verfahren auftretende Flüssigkeiten wie beispielsweise Blut, Lymphflüssigkeit, cytosolische Flüssigkeiten, oder sonstige im Körper vorkommende Flüssigkeiten, oder bei "in vitro"-Verfahren üblicherweise eingesetzte Flüssigkeiten oder Puffer. Solche Flüssigkeiten und Puffer sind einem Fachmann bekannt.

Der Injektionspuffer kann weitere Komponenten enthalten, bspw. Zucker (Mono-Di-Tri- oder Polysaccharide), insbesondere Glucose oder Mannitol. In einer bevorzugten Ausführungsform werden in dem zur erfindungsgemäßen Verwendung eingesetzten Injektionspuffer jedoch keine Zucker vorliegen. Auch ist es bevorzugt, dass der erfindungsgemäß eingesetzte Puffer gerade keine ungeladenen Komponenten, wie beispielsweise Zucker enthält. Typischerweise enthält der erfindungsgemäß eingesetzte Puffer ausschließlich Metall-Kationen, insbesondere aus der Gruppe der Alkali- oder Erdalkalimetalle, und Anionen, insbesondere die vorbezeichneten Anionen.

Der pH-Wert des Injektionspuffers liegt vorzugsweise zwischen 1 und 8,5, vorzugsweise zwischen 3 und 5, stärker bevorzugt zwischen 5,5 und 7,5, insbesondere zwischen 5,5 und 6,5. Ggf. kann der Injektionspuffer auch ein Puffersystem enthalten, das den Injektionspuffer auf einen gepufferten pH-Wert festlegt. Bspw. kann es sich um ein Phosphatpuffer-System, HEPES oder Na2HPO4/NaH2PO4 handeln. Ganz besonders bevorzugt ist jedoch der erfindungsgemäß verwendete Injektionspuffer dann, wenn er keines der vorgenannten Puffersysteme enthält oder überhaupt kein Puffersystem aufweist.

Der erfindungsgemäß verwendete Injektionspuffer enthält, wie zuvor beschrieben, Salze von Natrium, Calcium und Kalium, wobei Natrium und Kalium im Injektionspuffer typischerweise als monovalente Kationen ($Na^+$, $K^+$) vorliegen, und Calcium als divalentes Kation ($Ca^{2+}$) vorliegt. Gemäß einer bevorzugten Ausführungsform können zusätzlich zu diesen oder alternativ dazu im erfindungsgemäß verwendeten Injektionspuffer enthaltenen monovalenten und divalenten Kationen divalente Kationen, insbesondere aus der Gruppe der Erdalkalimetalle, wie beispielsweise Magnesium ($Mg^{2+}$), oder auch Eisen ($Fe^{2+}$), und monovalente Kationen, insbesondere aus der Gruppe der Alkalimetalle, wie beispielsweise Lithium ($Li^+$), enthalten sein. Diese monovalenten Kationen liegen bevorzugt in Form ihrer Salze vor, z.B. in Form von Halogeniden, z.B. Chloriden, Iodiden oder Bromiden, in Form ihrer Hydroxide, Carbonate, Hydrogencarbonate oder Sulfate vor. Beispielhaft sollen hier für das Lithiumsalz $LiCl$, $LiI$, $LiBr$, $Li_2CO_3$, $LiHCO_3$, $Li_2SO_4$, für das Magnesiumsalz $MgCl_2$, $MgI_2$, $MgBr_2$, $MgCO_3$, $MgSO_4$, und $Mg(OH)_2$ und für das Eisensalz $FeCl_2$, $FeBr_2$, $FeI_2$, $FeF_2$, $Fe_2O_3$, $FeCO_3$, $FeSO_4$, $Fe(OH)_2$. Umfasst werden ebenfalls sämtliche Kombinationen von di- und/oder monovalenten Kationen, wie zuvor beschrieben. So sind erfindungsgemäß eingesetzte Injektionspuffer umfasst, die nur divalente, nur monovalente oder di- und monovalente Kationen enthalten. Ebenfalls erfindungsgemäß umfasst sind Injektionspuffer, die lediglich eine Sorte di- oder monovalente Kationen enthalten, insbesondere bevorzugt z.B. lediglich $Ca^{2+}$-Kationen bzw. ein Salz davon, z.B. $CaCl_2$.

Bevorzugt ist hierbei, dass die voranstehend für $Ca^{2+}$ (als divalentes Kation) und $Na^{1+}$ (als monovalentes Kation) vorgegebenen Molaritäten (also typischerweise Konzentrationen von mindestens 50 mM $Na^+$, mindestens 0,01 mM $Ca^{2+}$ und mindestens 3 mM $K^+$) im Injektionspuffer auch dann berücksichtigt werden, wenn tw. oder vollständig anstelle von $Ca^{2+}$ bzw. $Na^{1+}$ ein anderes di- bzw. monovalentes Kationandere oder andere di- bzw. monovalente Kationen, insbesondere andere Kationen aus der Gruppe der Erdalkalimetalle bzw. Alkalimetalle, im erfindungsgemäß zur Herstellung der Injektionslösung verwendeten Injektionspuffer zum Einsatz kommen. Zwar können $Ca^{2+}$ bzw. $Na^{1+}$, wie vorstehend erwähnt, vollständig durch jeweils andere di- bzw. monovalente Kationen im erfindungsgemäß verwendeten Injektionspuffer ersetzt sein, bspw. auch durch eine Kombination von anderen divalenten Kationen (anstelle von $Ca^{2+}$) und/oder eine Kombination von anderen monovalenten Kationen (anstelle von $Na^{1+}$) (insbesondere eine Kombination von anderen divalenten Kationen aus der Gruppe der Erdalkalimetalle bzw. von anderen monovalenten Kationen aus der Gruppe der Alkalimetalle), jedoch ist es bevorzugt, $Ca^{2+}$ bzw. $Na^{1+}$ allenfalls tw. zu ersetzen, d.h. mindestens 20 %, vorzugsweise mindestens 40%, noch stärker mindestens 60% und noch stärker bevorzugt mindestens 80% der jeweiligen Gesamtmolaritäten der mono- bzw. divalenten Kationen im Injektionspuffer durch $Ca^{2+}$ bzw. $Na^{1+}$ zu besetzen.

Die Herstellung des Injektionspuffers erfolgt vorzugsweise bei Raumtemperatur (25°C) und atmosphärischem Luftdruck. Die Herstellung kann nach jedem beliebigen Verfahren aus dem Stand der Technik erfolgen. Vorzugsweise werden die enthaltenen Ionen bzw. Salze in wässriger Lösung verdünnt, wobei die jeweiligen Konzentrationsverhältnisse gemäß den jeweiligen Voraussetzungen (Wirtsorganismus, insbesondere Säugetier, dem die RNA-Injektionslösung injiziert wird, Gesundheitszustand, Alter etc. des Wirtsorganismus sowie Voraussetzungen der Löslichkeit und Interferenz der Komponenten, Reaktionstemperatur, -zeit, usw.) zu wählen sind.

Die Konzentrationen der in dem wässrigen Injektionspuffer enthaltenen Komponenten Natrium-, Calcium- und Chlorid-Ionen sowie Kalium-Ionen und ggf. Laktat (siehe nachfolgende Ausführungsformen) hängt insbesondere von deren Löslichkeit in Wasser, der Interferenz der Komponenten untereinander, aber auch von Reaktionstemperatur und -druck bei Herstellen des Injektionspuffers bzw. der RNA-Injektionslösung ab.

Der gemäß der vorliegenden Erfindung verwendete Injektionspuffer basiert auf einer wässrigen Lösung, d.h. aus einer Lösung bestehend aus Wasser und den erfindungsgemäß für die Injektionslösung verwendeten Salzen und ggf. Laktat. Die Salze der oben genannten monovalenten oder divalenten Kationen können in einer solchen wässrigen Lösung gegebenenfalls schwer- oder auch nicht-löslich sein. Das Maß der Löslichkeit der jeweiligen Salze lässt sich dabei aus dem Löslichkeitsprodukt errechnen. Verfahren zur genauen Bestimmung der Löslichkeit und des Löslichkeitsproduktes sind einem Fachmann bekannt. Diese wässrige Lösung kann bis zu 30 Mol % der in der Lösung enthaltenen Salze, vorzugsweise bis zu 25 Mol%, bevorzugt bis zu 20 Mol %, weiterhin bevorzugt bis zu 15 Mol %, stärker bevorzugt bis zu 10 Mol %, noch stärker bevorzugt bis zu 5 Mol %, ebenfalls stärker bevorzugt bis zu 2 Mol % nicht bzw. schwer lösliche Salze enthalten. Als schwer löslich im Sinne der vorliegenden Erfindung gelten solche Salze, deren Löslichkeitsprodukt < $10^{-4}$ ist. Als leicht löslich gelten solche Salze, deren Löslichkeitsprodukt > $10^{-4}$ ist.

Die Löslichkeit eines Salzes bzw. Ions oder Ionenverbindung in Wasser hängt von seiner Gitterenergie und der Hydratationsenergie und unter Berücksichtigung auftretender Entropieeffekte ab. Man spricht auch von dem Löslichkeitsprodukt, genauer, dem Gleichgewicht, das sich bei Lösung eines Salzes bzw. Ions oder Ionenverbindung in Wasser einstellt. Das Löslichkeitsprodukt wird allgemein als Produkt der Konzentrationen der Ionen in der gesättigten Lösung eines Elektrolyten definiert. Beispielsweise sind Alkalimetalle (wie z.B. $Na^+$, $K^+$) in Wasser in höheren Konzentrationen löslich als Erdalkalimetallsalze (wie z.B. $Ca^{2+}$-Salze), d.h. sie haben ein größeres Löslichkeitsprodukt. D.h., dass die in der wässrigen Lösung des erfindungsgemäß eingesetzten Injektionspuffers enthaltenen Kalium- und NatriumSalze leichter löslich sind als die enthaltenen Calcium-Salze. Daher muss u.a. die Interferenz zwischen den Kalium-, Natrium- und Calcium-Salze bei der Bestimmung der Konzentration dieser Ionen berücksichtigt werden.

Bevorzugt ist eine erfindungsgemäße Verwendung, bei welcher der eingesetzte Injektionspuffer von 50 mM bis 800 mM, bevorzugt von 60 mM bis 500 mM, stärker bevorzugt von 70 mM bis 250 mM, insbesondere bevorzugt 60 mM bis 110 mM Natriumchlorid (NaCl), von 0,01 mM bis 100 mM, bevorzugt von 0,5 mM bis 80 mM, stärker bevorzugt von 1,5 mM bis 40 mM Calciumchlorid ($CaCl_2$) und von 3 mM bis 500 mM, bevorzugt von 4 mM bis 300 mM, stärker bevorzugt von 5 mM bis 200 mM Kaliumchlorid (KCl) enthält.

Als weitere Anionen können neben den vorgenannten anorganischen Anionen, bspw. Halogeniden, Sulfaten oder Carbonaten, auch organische Anionen auftreten. Hierunter sind zu nennen Succinat, Lactobionat, Laktat, Malat, Maleonat etc., die auch kombiniert enthalten sein können. Vorzugsweise enthält ein Injektionspuffer zur erfindungsgemäßen Verwendung Laktat, insbesondere bevorzugt weist ein solcher Injektionspuffer, sofern ein organisches Anion enthalten ist, ausschließlich Laktat als organisches Anion auf. Laktat im Sinne der Erfindung kann jedes beliebige Laktat sein, beispielsweise L-Laktat und D-Laktat. Als Laktatsalze treten im Zusammenhang mit der vorliegenden Erfindung typischerweise Natriumlaktat und/oder Calciumlaktat auf, insbesondere dann, wenn der Injektionspuffer lediglich Na⁺ als monovalentes Kation aufweist und Ca²⁺ als divalentes Kation.

In einer bevorzugten Ausführungsform der erfindungsgemäßen Verwendung enthält ein erfindungsgemäß eingesetzter Injektionspuffer bevorzugt von 15 mM bis 500 mM, stärker bevorzugt von 15 mM bis 200 mM, und noch stärker bevorzugt von 15 mM bis 100 mM Laktat.

Erfindungsgemäß wurde festgestellt, dass die Verwendung eines Injektionspuffers mit den vorstehend beschriebenen Komponenten, optional mit oder ohne Laktat (nachfolgend: "RL-Injektionspuffer", wenn die Komponente Laktat nicht enthalten ist, bzw. "RL-Injektionspuffer mit Laktat", wenn die Komponente Laktat enthalten ist) für RNA-Injektionslösungen (d.h. Injektionslösungen, die RNA enthalten und für die Injektion dieser RNA geeignet sind) sowohl den Transfer als auch die Translation der RNA in die/den Zellen/Gewebe eines Wirtsorganismus (Säugetier) signifikant erhöht im Vergleich zu im Stand der Technik herkömmlich verwendeten Injektionspuffern.

Eine Lösung mit den vorstehend bezeichneten Komponenten Natriumchlorid (NaCl), Calciumchlorid (CaCl₂), Laktat, insbesondere Natriumlaktat, und weiterhin Kaliumchlorid (KCl) ist auch als "Ringer-Lösung" oder "Ringer-Laktat" bekannt. Ringer-Laktat ist eine kristalloide Vollelektrolytlösung, die als Volumenersatz und als Trägerlösung, beispielsweise für kompatible Arzneimittel, verwendet wird. Beispielsweise wird Ringer-Laktat bei Flüssigkeits- und Elektrolytverlust (durch Erbrechen, Durchfall, Darmverschluss oder Verbrennung) als primäres Volumenersatzmittel, insbesondere bei Säuglingen und Kleinkindern, und zum Offenhalten von peripheren und/oder zentralen Venenzugängen verwendet. Die erfindungsgemäße Verwendung von Ringer-Laktat als Injektionspuffer in einer RNA-Injektionslösung wird im Stand der Technik allerdings nicht beschrieben.

RNA kann jede beliebige RNA, beispielsweise mRNA, tRNA, rRNA, siRNA, einzel- oder doppelsträngige RNA, Heteroduplex-RNA, etc. sein. Die erfindungsgemäß verwendete RNA ist mRNA und kann für jedes beliebige Protein von Interesse kodieren. Vorzugsweise handelt es sich bei der erfindungsgemäß verwendeten mRNA um nackte mRNA.

Unter nackter mRNA im Sinne der Erfindung ist eine mRNA zu verstehen, die nicht komplexiert, bspw. mit polykationen Molekülen, vorliegt. Nackte mRNA kann einzelsträngig, aber auch doppelsträngig also als Sekundärstruktur, z. B. als sogenannte "hair pin structure" bzw. Haarnadelstruktur, vorliegen. Solche Doppelstrang-Formen treten vor allem innerhalb der nackten mRNA auf, wenn komplementäre Ribonucleotid-Abfolgen in dem Molekül vorliegen.

Erfindungsgemäß kann die mRNA, jedoch auch komplexiert vorliegen. Durch eine solche Komplexierung/Kondensierung der mRNA der Erfindung kann der wirksame Transfer der RNA in die zu behandelnden Zellen bzw. das zu behandelnde Gewebe des zu behandelnden Organismus dadurch verbessert werden, dass die mRNA mit einem (poly)kationischen Polymer, Peptid oder Protein assoziiert oder daran gebunden wird. Vorzugsweise wird eine solche mRNA mit mindestens einem kationischen oder polykationischen Agens komplexiert oder kondensiert. Bevorzugt handelt es sich bei einem solchen kationischen oder poykationischen Agens um ein Agens, das aus der Gruppe bestehend aus Protamin, Poly-L-Lysin, Poly-L-Arginin, Nucleolin, Spermin und Histonen oder Derivaten von Histonen oder Protaminen ausgewählt wird. Insbesondere bevorzugt ist die Verwendung von Protamin als polykationisches, Nukleinsäure-bindendes Protein. Diese Vorgehensweise zur Stabilisierung der mRNA wird beispielsweise in EP-A-1083232 beschrieben.

Die erfindungsgemäß eingesetzte mRNA kann weiterhin modifiziert vorliegen. Diese Modifikationen dienen vor allem der Erhöhung der Stabilität der mRNA. Vorzugsweise weist die mRNA eine oder mehrere (natürlich auftretende oder nichtnative) Modifikationen, insbesondere chemische Modifikationen, auf, die beispielsweise zur Erhöhung der Halbwertszeit der RNA im Organismus beitragen oder Translationseffizienz der mRNA im Cytosol gegenüber der Translationseffizienz nicht modifizierter mRNA im Cytosol verbessern. Vorzugsweise wird die Translationseffizienz durch eine erfindungsgemäße Modifikation um mindestens 10%, bevorzugt mindestens 20%, ebenfalls bevorzugt um mindestens 40%, stärker bevorzugt um mindestens 50%, weiterhin stärker bevorzugt um mindestens 60%, ebenfalls stärker bevorzugt um mindestens 75%, am meisten bevorzugt um mindestens 85%, am meisten bevorzugt um mindestens 100% gegenüber der Translationseffizienz nicht modifizierter mRNA im Cytosol verbessern Beispielsweise kann der G/C-Gehalt des kodierenden Bereichs einer modifizierten mRNA gegenüber dem G/C-Gehalt des kodierenden Bereichs der entsprechenden Wildtyp-mRNA erhöht werden, wobei die kodierte Aminosäuresequenz der modifizierten mRNA gegenüber der kodierten Aminosäuresequenz der Wildtyp-mRNA vorzugsweise unverändert bleibt. Diese Modifikation beruht auf der Tatsache, dass für die effiziente Translation einer mRNA die Sequenzabfolge des zu translatierenden Bereichs der mRNA wesentlich ist. Bedeutungsvoll ist hier die Zusammensetzung und die Abfolge der verschiedenen Nukleotide. Insbesondere sind Sequenzen mit hohen G (Guanosin)/C (Cytosin)-Gehalt stabiler als Sequenzen mit einem hohen A (Adenosin)/U (Uracil)-Gehalt. Daher ist es sinnvoll unter Beibehaltung der translatierten Aminosäureabfolge die Codons gegenüber der Wildtyp-mRNA derart zu variieren, dass sie vermehrt G/C-Nukleotide beinhalten. Aufgrund der Tatsache, dass mehrere Codons für ein und dieselbe Aminosäure kodieren (sog. "Degeneration des genetischen Codes"), können die für die Stabilität günstigsten Codons, vorzugsweise mit maximalem G/C-Gehalt, ermittelt werden. Im Ergebnis weist ein RNA im Injektionspuffer bevorzugt einen mindestens 30%, stärker bevorzugt einen mindestens 50%, noch stärker bevorzugt einen mindestens 70%, stärker bevorzugt 80% erhöhten G/C-Gehalt, bezogen auf den maximalen G/C-Gehalt (also den G/C-Gehalt nach Modifikation aller potentiellen Tripletts im codierenden Bereich ohne Veränderung der codierten Aminosäuresequenz unter Nutzung des Degeneriertheit des genetischen Codes, ausgehend von der nativen Sequenz, mit dem Ziel der Maximierung des G/C-Gehalts) und am stärksten bevorzugt den maximalen G/C-Gehalt auf, wobei der maximale G/C-Gehalt durch jene Sequenz vorgegeben wird, deren G/C-Gehalt maximiert ist, ohne daß hierdurch die kodierte Aminosäuresequenz verändert wird.

In Abhängigkeit von der durch die modifizierte mRNA zu kodierenden Aminosäure sind unterschiedliche Möglichkeiten zur Modifikation der mRNA-Sequenz gegenüber der Wildtyp-Sequenz möglich. Im Fall von Aminosäuren, die durch Codons kodiert werden, die ausschließlich G- oder C-Nukleotide enthalten, ist keine Modifikation des Codons erforderlich. Beispiele hierfür sind Codons für Pro (CCC oder CCG), Arg (CGC oder CGG), Ala (GCC oder GCG) und Gly (GGC oder GGG).

Dagegen können Codons, die A- und/oder U-Nukleotide enthalten, durch Substitution anderer Codons, welche die gleichen Aminosäuren kodieren, jedoch kein A und/oder U enthalten, verändert werden. Beispiele hierfür sind:

- Codons für Pro können von CCU oder CCA zu CCC oder CCG verändert werden;
- Codons für Arg können von CGU oder CGA oder AGA oder AGG zu CGC oder CGG verändert werden;
- Codons für Ala können von GCU oder GCA zu GCC oder GCG verändert werden;
- Codons für Gly können von GGU oder GGA zu GGC oder GGG verändert werden.

In einigen Fällen können A- bzw. U-Nukleotide zwar nicht aus den Codons eliminiert werden, jedoch ist es möglich, den A- und U-Gehalt zu verringern, indem Codons verwendet werden, die einen geringeren Anteil A- und/oder U-Nukleotide enthalten. Beispiele hierfür sind:
die Codons für Phe können von UUU zu UUC verändert werden;

- Codons für Leu können von UUA, UUG, CUU oder CUA zu CUC oder CUG verändert werden;
- Codons für Ser können von UCU oder UCA oder AGU zu UCC, UCG oder AGC verändert werden;
- das Codon für Tyr kann von UAU zu UAC verändert werden;
- das Codon für Cys kann von UGU zu UGC verändert werden;
- das Codon His kann von CAU zu CAC verändert werden;
- das Codon für Gln kann von CAA zu CAG verändert werden;
- Codons für Ile können von AUU oder AUA zu AUC verändert werden;
- Codons für Thr können von ACU oder ACA zu ACC oder ACG verändert werden;
- das Codon für Asn kann von AAU zu AAC verändert werden;
- das Codon für Lys kann von AAA zu AAG verändert werden;
- Codons für Val können von GUU oder GUA zu GUC oder GUG verändert werden;
- das Codon für Asp kann von GAU zu GAC verändert werden;
- das Codon für Glu kann von GAA zu GAG verändert werden,
- das Stop-Codon UAA kann zu UAG oder UGA verändert werden.

Die vorstehend aufgeführten Substitutionen können einzeln aber auch in allen möglichen Kombinationen zur Erhöhung des G/C-Gehalts der modifizierten mRNA gegenüber der Wildtyp-mRNA (der ursprünglichen Sequenz) verwendet werden. Bevorzugt werden beispielsweise Kombinationen der vorstehenden Substitutionsmöglichkeiten verwendet:

- Substitution aller in der ursprünglichen Sequenz (Wildtyp-mRNA) für Thr kodierenden Codons zu ACC (oder ACG) und Substitution aller ursprünglich für Ser kodierenden Codons zu UCC (oder UCG oder AGC);
- Substitution aller in der ursprünglichen Sequenz für Ile kodierenden Codons zu AUC und Substitution aller ursprünglich für Lys kodierenden Codons zu AAG und Substitution aller ursprünglich für Tyr kodierenden Codons zu UAC;
- Substitution aller in der ursprünglichen Sequenz für Val kodierenden Codons zu GUC (oder GUG) und Substitution aller ursprünglich für Glu kodierenden Codons zu GAG und Substitution aller ursprünglich für Ala kodierenden Codons zu GCC (oder GCG) und Substitution aller ursprünglich für Arg kodierenden Codons zu CGC (oder CGG);
- Substitution aller in der ursprünglichen Sequenz für Val kodierenden Codons zu GUC (oder GUG) und Substitution aller ursprünglich für Glu kodierenden Codons zu GAG und Substitution aller ursprünglich für Ala kodierenden Codons zu GCC (oder GCG) und Substitution aller ursprünglich für Gly kodierenden Codons zu GGC (oder GGG) und Substituion aller ursprünglich für Asn kodierenden Codons zu AAC;

- Substitution aller in der ursprünglichen Sequenz für Val kodierenden Codons zu GUC (oder GUG) und Substitution aller usprünglich für Phe kodierenden Codons zu UUC und Substitution aller ursprünglich für Cys kodierenden Codons zu UGC und Substitution aller ursprünglich für Leu kodierenden Codons zu CUG (oder CUC) und Substitution aller ursprünglich für Gln kodierenden Codons zu CAG und Substitution aller ursprünglich für Pro kodierenden Codons zu CCC (oder CCG);

usw.

Im Falle einer Veränderung des G/C-Gehalt des für das Protein kodierenden Bereichs der modifizierten mRNA wird dieser um mindestens 7%-Punkte, stärker bevorzugt um mindestens 15%-Punkte, ebenfalls stärker bevorzugt um mindestens 20%-Punkte, noch stärker bevorzugt um mindestens 30%-Punkte gegenüber dem G/C-Gehalt des kodierten Bereichs der für das Protein kodierenden Wildtyp-mRNA erhöht sein. Besonders bevorzugt ist es in diesem Zusammenhang, den G/C-Gehalt der modifizierten mRNA, insbesondere in dem für das Protein kodierenden Bereich, im Vergleich zur Wildtyp-Sequenz maximal zu erhöhen.

Weiterhin bevorzugt ist eine Erhöhung des A/U-Gehalts in der Umgebung der Ribosomen-Bindungsstelle der modifizierten mRNA des gegenüber dem A/U-Gehalt in der Umgebung der Ribosomen-Bindungsstelle der entsprechenden Wildtyp-mRNA. Diese Modifikation erhöht die Effizienz der Ribosomen-Bindung an die mRNA. Eine wirksame Bindung der Ribosomen an die Ribosomen-Bindungsstelle (Kozak-Sequenz: GCCGCCACCAUGG, das AUG bildet das Startcodon) bewirkt wiederum eine effiziente Translation der mRNA. Die Erhöhung besteht in der Einführung mindestens einer zusätzlichen A/U-Einheit, typischerweise von mindestens 3 im Bereich der Bindungsstelle, also -20 bis +20 vom A des AUG-Startcodons.

Eine ebenfalls bevorzugte Modifikation betrifft eine mRNA, bei der der kodierende Bereich und/oder der 5'- und/oder 3'-nicht-translatierte Bereich der modifizierten mRNA gegenüber der Wildtyp-mRNA derart verändert ist, dass er keine destabilisierenden Sequenzelemente enthält, wobei die kodierte Aminosäuresequenz der modifizierten mRNA gegenüber der Wildtyp-mRNA vorzugsweise nicht verändert ist. Es ist bekannt, dass beispielsweise in den Sequenzen eukaryotischer mRNAs destabilisierende Sequenzelemente (DSE) auftreten, an welche Signalproteine binden und den enzymatischen Abbau der mRNA *in vivo* regulieren. Daher können zur weiteren Stabilisierung der erfindungsgemäßen modifizierten mRNA gegebenenfalls im für das Protein kodierenden Bereich ein oder mehrere derartige Veränderungen gegenüber dem entsprechenden Bereich der Wildtyp-mRNA vorgenommen werden, so dass dort keine bzw. im wesentlichen keine destabilisierenden Sequenzelemente enthalten sind. Durch derartige Veränderungen können erfindungsgemäß ebenfalls in den nicht-translatierten Bereichen (3'- und/oder 5'-UTR) vorhandene DSE aus der mRNA eliminiert werden.

Derartige destabilisierende Sequenzen sind bspw. AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674) sowie Sequenzmotive, die von Endonucleasen erkannt werden (z.B. Binder et al., EMBO J. 1994, 13: 1969 bis 1980).

Ebenfalls bevorzugt ist eine modifizierte mRNA, die eine 5'-Cap-Struktur zur Stabilisierung aufweist. Beispiele von Cap-Strukturen, die erfindungsgemäß verwendet werden können, sind m7G(5')ppp (5'(A,G(5')ppp(5')A und G(5')ppp(5')G.

Ferner ist es bevorzugt, dass die modifizierte mRNA einen Poly(A)-Schwanz, vorzugsweise von mindestens 25 Nukleotiden, stärker bevorzugt von mindestens 50 Nukleotiden, noch stärker bevorzugt von mindestens 70 Nukleotiden, ebenfalls stärker bevorzugt von mindestens 100 Nukleotiden, am stärksten bevorzugt von mindestens 200 Nukleotiden aufweist.

Ebenfalls bevorzugt weist die modifizierte mRNA mindestens eine IRES- und/oder mindestens eine 5'- und/oder 3'-Stabilisierungssequenz auf. Erfindungsgemäß können in die modifizierte mRNA eine oder mehrere sog. IRES (engl. "internal ribosomal entry side") eingefügt werden. Eine IRES kann so als alleinige Ribosomen-Bindungsstelle fungieren, sie kann jedoch auch zur Bereitstellung einer mRNA dienen, die mehrere Proteine, Peptide bzw. Polypeptide kodiert, die unabhängig voneinander durch die Ribosomen translatiert werden sollen ("multicistronische mRNA"). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassische-Schweinefieber-Viren (CSFV), Murines-Leukoma-Virus (MLV), Simean-Immundefizienz-Viren (SIV) oder Cricket-Paralysis-Viren (CrPV).

Weiterhin bevorzugt weist eine modifizierte mRNA mindestens eine 5'- und/oder 3'-Stabilisierungssequenz auf. Diese Stabilisierungssequenzen in den 5'- und/oder 3'-nicht-translatierten Bereichen bewirken eine Erhöhung der Halbwertszeit der mRNA im Cytosol. Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des Globingens, bspw. von *Homo sapiens* oder *Xenopus laevis,* genannt werden. Ein anderes Beispiel einer Stabilisierungssequenz weist die allgemeine Formel

(C/U)CCAN$_x$CCC(U/A)Py$_x$UC(C/U)CC auf, die im 3'UTR der sehr stabilen mRNA enthalten ist, die für Globin, (I)-Collagen, 15-Lipoxygenase oder für Tyrosin-Hydroxylase kodiert (vgl. Holcik et al., Proc. Natl. Acad. Sci. USA 1997, 94: 2410 bis 2414). Selbstverständlich können derartige Stabilisierungssequenzen einzeln oder in Kombination miteinander, und auch in Kombination mit anderen, einem Fachmann bekannten Stabilisierungssequenzen verwendet werden.

In einer bevorzugten Ausführungsform der vorliegenden Erfindung weist die modifizierte mRNA mindestens ein Analoges natürlich vorkommender Nukleotide auf. Dieses/diese Analog/Analoga dient/dienen der weiteren Stabilisierung der modifizierten mRNA, wobei dies auf der Tatsache beruht, dass die in den Zellen vorkommenden RNA-abbauenden Enzyme als Substrat vorzugsweise natürlich vorkommende Nukleotide erkennen. Durch Einfügen von Nukleotid-Analoga in die mRNA wird daher der mRNA-Abbau erschwert, wobei die Auswirkung auf die Translationseffizienz bei Einfügen dieser Analoga, insbesondere in den kodierenden Bereich der mRNA, einen positiven oder negativen Effekt haben kann. In einer keineswegs abschließenden Aufzählung können als Beispiele erfindungsgemäß verwendbarer Nukleotidanaloga Phosphoramidate, Phosphorthioate, Peptidnukleotide, Methylphosphonate, 7-Deazaguanosin, 5-Methylcytosin und Inosin verwendet werden. Die Herstellung derartiger Analoga sind einem Fachmann bekannt, bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642. Derartige Analoga können sowohl in nicht-translatierten als auch in translatierten Bereichen der modifizierten mRNA vorkommen.

In einer weiteren bevorzugten Ausführungsform der vorliegenden Erfindung weist die modifizierte mRNA zusätzlich eine für ein Signalpeptid kodierende Sequenz auf. Diese für ein Signalpeptid kodierende Sequenz ist bevorzugt 30 bis 300 Basen lang, kodierend für 10 bis 100 Aminosäuren. Stärker bevorzugt ist die für ein Signalpeptid kodierende Sequenz 45 bis 180 Basen lang, die für 15 bis 60 Aminosäuren kodieren. Beispielhaft können zur Modifikation der erfindungsgemäß eingesetzten mRNA folgende in Tabelle 1 genannten Sequenzen eingesetzt werden. Umfasst sind dabei auch solche der in Tabelle 1 genannten Sequenzen, die 1-20, bevorzugt 1-10 und am stärksten bevorzugt 1-5 Basenaustausche gegen A, T, C oder G in im Vergleich zu einer der in Tabelle 1 genannten Sequenz aufweisen.

Tab. 1: Beispiele von Signalpeptidsequenzen: Aminosäuresequenzen , die durch das erste Exon von MHC Klasse I oder MHC Klasse II Genen kodiert werden, und Myelin oligodendroocyte glycoprotein.

| Name der Signalsequenz | Sequenz (Peptid- und Nukleotidsequenz) |
| --- | --- |
| HLA-B*07022 | MLVMAPRTVLLLLSAALALTETWAG<br>RNA Sequenz (GC enriched)<br>AUG CUG GUG AUG GCC CCG CGG ACC GUC CUC CUG CUG CUG AGC GCG GCC CUG GCC CUG ACG GAG ACC UGG GCC GGC |
| HLA-A*3202 | MAVMAPRTLLLLLLGALALTQTWAG<br>RNA Sequenz (GC enriched)<br>AUG GCC GUG AUG GCG CCG CGG ACC CUG CUC CUG CUG CUG CUG GGC GCC CUG GCC CUC ACG CAG ACC UGG GCC GGG |
| HLA-A*01011 | MAVMAPRTLLLLLSGALALTQTWAG<br>AUG GCC GUG AUG GCG CCG CGG ACC CUG CUC CUG CUG CUG AGC GGC GCC CUG GCC CUG ACG CAG ACC UGG GCC GGG |
| HLA-A*0102 | MAVMAPRTLLLLLSGALALTQTWAG<br>AUG GCC GUG AUG GCG CCG CGG ACC CUG CUC CUG CUG CUG AGC GGC GCC CUG GCC CUG ACG CAG ACC UGG GCC GGG |
| HLA-A*0201 | MAVMAPRTLVLLLSGALALTQTWAG<br>AUG GCC GUG AUG GCG CCG CGG ACC CUG GUC CUC CUG CUG AGC GGC GCC CUG GCC CUG ACG CAG ACC UGG GCC GGG |
| HLA-A*0301 | MAVMAPRTLLLLLSGALALTQTWAG |

(fortgesetzt)

| Name der Signalsequenz | Sequenz (Peptid- und Nukleotidsequenz) |
|---|---|
| | AUG GCC GUG AUG GCG CCG CGG ACC CUG CUC CUG CUG CUG AGC GGC GCC CUG GCC CUG ACG CAG ACC UGG GCC GGG |
| HLA-A*1101 | MAVMAPRTLLLLLSGALALTQTWAG |
| | AUG GCC GUG AUG GCG CCG CGG ACC CUG CUC CUG CUG CUG AGC GGC GCC CUG GCC CUG ACG CAG ACC UGG GCC GGG |
| HLA-B*070201 | MLVMAPRTVLLLLSAALALTETWAG |
| | AUG CUG GUG AUG GCC CCG CGG ACC GUC CUC CUG CUG CUG AGC GCG GCC CUG GCC CUG ACG GAG ACC UGG GCC GGC |
| HLA-B*2702 | MRVTAPRTLLLLLWGAVALTETWAG |
| | AUG CGG GUG ACC GCC CCG CGC ACG CUG CUC CUG CUG CUG UGG GGC GCG GUC GCC CUG ACC GAG ACC UGG GCC GGG |
| HLA-Cw*010201 | MRVMAPRTULLLSGALALTETWACS |
| | AUG CGG GUG AUG GCC CCG CGC ACC CUG AUC CUC CUG CUG AGC GGC GCG CUG GCC CUG ACG GAG ACC UGG GCC UGC UCG |
| HLA-Cw*02021 | MRVMAPRTLLLLLSGALALTETWACS |
| | AUG CGG GUG AUG GCC CCG CGC ACC CUG CUC CUG CUG CUG AGC GGC GCG CUG GCC CUG ACG GAG ACC UGG GCC UGC UCG |
| HLA-E*0101 | MVDGTLLLLSSEALALTQTWAGSHS |
| | AUG GUG GAC GGC ACC CUG CUC CUG CUG AGC UCG GAG GCC CUG GCG CUG ACG CAG ACC UGG GCC GGG AGC CAC AGC |
| HLA-DRB1 | MVCLKLPGGSCMTALTVTLMVLSSPLALA |
| | AUG GUG UGC CUG AAG CUC CCG GGC GGG AGC UGC AUG ACC GCC CUG ACG GUC ACC CUG AUG GUG CUG UCG AGC CCC CUG GCG CUG GCC |
| HLA-DRA1 | MAISGVPVLGFFIIAVLMSAQESWA |
| | AUG GCC AUC AGC GGC GUG CCG GUC CUG GGG UUC UUC AUC AUC GCG GUG CUC AUG UCG GCC CAG GAG AGC UGG GCC |
| HLA-DR4 | MVCLKFPGGSCMAALTVTLMVLSSPLALA |
| | AUG GUG UGC CUG AAG UUC CCG GGC GGG AGC UGC AUG GCC GCG CUC ACC GUC ACG CUG AUG GUG CUG UCG AGC CCC CUG GCC CUG GCC |
| Myelin oligodendroocyte glycoprotein | MACLWSFSWPSCFLSLLLLLLLQLSCSYA |
| | AUG GCC UGC CUG UGG AGC UUC UCG UGG CCG AGC UGC UUC CUC AGC CUG CUG CUG CUG CUG CUC CUG CAG CUG AGC UGC AGC UAC GCG |

Einem Fachmann sind verschiedene Verfahren geläufig, die vorstehend beschriebenen Modifikationen vorzunehmen. Beispielsweise kann für die Substitution von Codons in der erfindungsgemäß eingesetzten, modifizierten mRNA im Falle kürzerer kodierender Bereiche, die gesamte mRNA chemisch unter Verwendung von Standardtechniken synthetisiert werden. Bevorzugt werden Substitutionen, Additionen oder Eliminierungen von Basen allerdings unter Verwendung einer DNA-Matrize zur Herstellung der modifizierten mRNA mit Hilfe von Techniken der gängigen zielgerichteten Mutagenese eingeführt (siehe z.B. Maniatis et al., Molecular Cloning: A Laboratory Manual, Cold Spring Harbor Laboratory Press, 3. Aufl., Cold Spring Harbor, NY, 2001). Bei einem solchen Verfahren wird zur Herstellung der mRNA ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nukleotidsequenz für die herzustellende mRNA und ein Terminationssignal für die *in vitro* Transkription folgen. Das DNA-Molekül, das die Matrize des herzustellenden RNA-Konstrukts bildet, kann durch fermentative Vermehrung und anschließende Isolierung als Teil eines in Bakterien replizierbaren Plasmids hergestellt werden. So kann unter Verwendung kurzer synthetischer DNA-Oligonukleotide, die an den entstehenden Schnittstellen kurze einzelsträngige Übergänge aufweisen, oder durch chemische Synthese hergestellte Gene, die gewünschte Nukleotidsequenz nach einem Fachmann geläufigen molekularbiologischen Methoden in ein geeignetes Plasmid kloniert werden (vgl. Maniatis et al., supra). Das DNA-Molekül wird dann aus dem Plasmid, in welchem es in einfacher oder mehrfacher Kopie vorliegen kann, durch Verdauung mit Restriktionsendonucleasen ausgeschnitten.

Die vorstehend beschriebenen Modifikationen der mRNA können im Sinne der Erfindung einzeln oder in Kombinationen miteinander auftreten. Ebenfalls kann/können eine oder mehrere Modifikation(en) mit der oben beschriebenen Komplexierung der mRNA kombiniert werden.

Die Erfindung hat die Steigerung des mRNA-Transfers und/oder der mRNA-Translation in einem Wirtsorganismus zum Ziel. Unter einem Wirtsorganismus im Sinne der Erfindung ist jeder Organismus zu verstehen, in dessen Zellen bzw. Gewebe mRNA transferiert werden kann, gefolgt von deren Translation. Ein Wirtsorganismus im Sinne der Erfindung ist insbesondere ein Säugetier, ausgewählt aus der Gruppe bestehend aus Maus, Ratte, Schwein, Rind, Pferd, Hund, Katze, Affe und insbesondere Mensch.

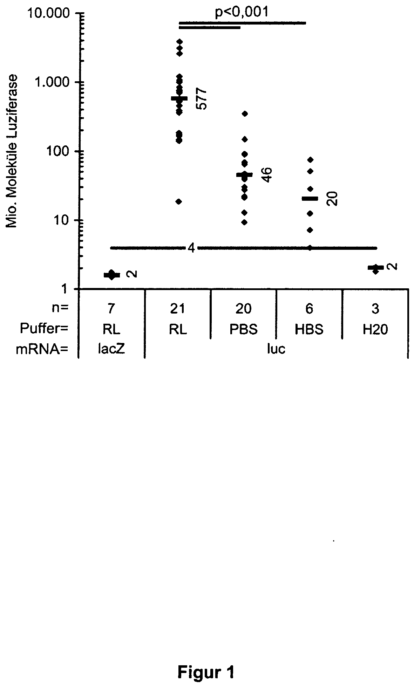

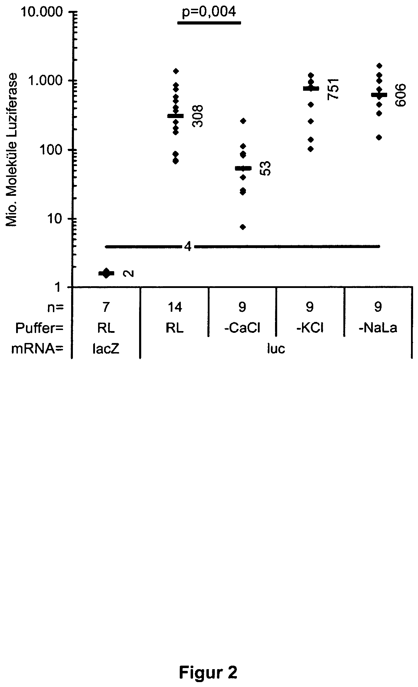

Mit der vorliegenden Erfindung wird gezeigt, dass in dem erfindungsgemäß eingesetzten RL-Injektionspuffer (mit oder ohne Laktat) verdünnte, Luziferase kodierende mRNA eine signifikant höhere Translationsrate ergibt, als mRNA, die in herkömmlich für RNA verwendeten Standardpuffern, wie HBS oder PBS, verdünnt wurde (siehe Figur 1). Weiterhin wird gezeigt, dass die Effizienz von Transfer und Translation von injizierter mRNA in starkem Maße von der Anwesenheit von Calcium-Ionen abhängig ist. In entsprechenden Vergleichsversuchen mit/ohne Calcium-Ionen im RL-Injektionspuffer (mit oder ohne Laktat) wurde festgestellt, dass die Abwesenheit von Calcium die Effizienz des mRNA-Transfers signifikant auf ein Level senkt, das mit dem der Standardpuffer PBS und HBS vergleichbar ist (siehe Figur 2).

Es wurde daher festgestellt, dass erstens ein erfindungsgemäß eingesetzter RL-Injektionspuffer (mit oder ohne Laktat) den mRNA-Transfer erheblich steigert, und dass dieser verbesserte mRNA-Transfer zweitens durch einen erfindungsgemäß eingesetzten RL-Injektionspuffer (mit oder ohne Laktat) mit einer hohen CalciumKonzentration von bis zu 100 mM noch um einen weiteren Faktor gesteigert wird.

Der erfindungsgemäß eingesetzte Injektionspuffer wird in Kombination mit mRNA in einer RNA-Injektionslösung verwendet. Ein weiterer Gegenstand der Erfindung ist daher eine RNA-Injektionslösung, enthaltend mRNA und einen Injektionspuffer, der bevorzugt mindestens 50 mM Natriumchlorid (NaCl), mindestens 0,01 mM Calciumchlorid ($CaCl_2$) und mindestens 3 mM Kaliumchlorid (KCl) enthält, zur Verwendung nach Anspruch 11 mit dem Ziel einer Steigerung des mRNA-Transfers und/oder der mRNA-Translation in Zellen. Bevorzugt wird eine erfindungsgemäße RNA-Injektionslösung, in welcher der Injektionspuffer mindestens 50 mM bis 800 mM, bevorzugt mindestens 60 mM bis 500 mM, stärker bevorzugt mindestens 70 mM bis 250 mM, insbesondere bevorzugt 60 mM bis 110 mM Natriumchlorid (NaCl), mindestens 0,01 mM bis 100 mM, bevorzugt mindestens 0,5 mM bis 80 mM, stärker bevorzugt mindestens 1,5 mM bis 40 mM Calciumchlorid ($CaCl_2$) und mindestens 3 mM bis 500 mM, bevorzugt mindestens 4 mM bis 300 mM, stärker bevorzugt mindestens 5 mM bis 200 mM Kaliumchlorid (KCl) enthält.

Der Injektionspuffer der erfindungsgemäßen RNA-Injektionslösung enthält vorzugsweise weiterhin Laktat. Bevorzugt enthält ein solcher Injektionspuffer der erfindungsgemäßen RNA-Injektionslösung mindestens 15 mM Laktat. Weiterhin bevorzugt wird eine erfindungsgemäße RNA-Injektionslösung, in welcher der Injektionspuffer von 15 mM bis 500 mM, bevorzugt von 15 mM bis 200 mM, stärker bevorzugt von 15 mM bis 100 mM Laktat enthält.

Die Herstellung der RNA-Injektionslösung kann nach jedem beliebigen Verfahren aus dem Stand der Technik erfolgen. Vorzugsweise wird die mRNA in dem RL-Injektionspuffer bzw. RL-Injektionspuffer mit Laktat verdünnt. Ebenfalls kann die mRNA als trockene mRNA (beispielsweise gefriergetrocknet) vorgegeben werden und der RNA-Injektionspuffer bzw. RL-Injektionspuffer mit Laktat hinzugegeben werden, optional unter Temperaturerhöhung, Rühren, Ultraschall etc., um das In-Lösung-gehen zu beschleunigen. Die jeweiligen Konzentrationsverhältnisse gemäß den jeweiligen Voraussetzungen (Wirtsorganismus, insbesondere Säugetier, dem die RNA-Injektionslösung injiziert wird, Gesundheitszustand, Alter etc. des Wirtsorganismus usw.) zu wählen sind.

Bei der mRNA in der erfindungsgemäßen RNA-Injektionslösung handelt es sich bevorzugt um nackte mRNA.

Wie beschrieben, ist die erfindungsgemäße RNA-Injektionslösung insbesondere zur Steigerung des RNA-Transfers und der RNA-Translation in einen/einem Wirtsorganismus einsetzbar.

Ein weiterer (nicht beanspruchter) Gegenstand ist demnach die Verwendung der vorstehend beschriebenen RNA-Injektionslösung zur Steigerung des RNA-Transfers und/oder der RNA-Translation in einen/einem Wirtsorganismus.

Ebenfalls wurde die Dosierung (hinsichtlich Menge und Dauer für insbesondere klinische Anwendungen) der zu transferierenden mRNA in RL-Injektionspuffer (mit oder ohne Laktat) untersucht. Diese Untersuchungen ergaben einen Anstieg der Luziferase-Expression mit ansteigenden Mengen an mRNA bis zu 0,1 $\mu$g (in 100 $\mu$l Injektionsvolumen) bei Mäusen und bis zu 3 mg (in 150 $\mu$l Injektionsvolumen) beim Menschen. Die Translation von mRNA erfolgt transient und wird konsequent reguliert, so dass für eine anhaltende, gleichmäßige Expression des Fremdmoleküls (Protein) eine Wiederholungsinjektion, abhängig von diversen Faktoren, wie dem zu exprimierenden Fremdmolekühl und der beabsichtigten Wirkung, dem Organismus, der die Injektion erhält, sowie dessen (Gesundheits-)zustand etc. ungefähr alle drei Tage, jedoch auch alle zwei Tage oder auch täglich vorgenommen werden sollte. Die Menge der mRNA kann - ebenfalls abhängig von verschiedenen, u. a. den vorstehend erwähnten, Faktoren - von 0,01 $\mu$g bis 1.000 $\mu$g, vorzugsweise von 1 $\mu$g bis 800 $\mu$g, ebenfalls bevorzugt von 2 $\mu$g bis 500 $\mu$g, stärker bevorzugt von 5 $\mu$g bis 100 $\mu$g, noch stärker bevorzugt von 10 $\mu$g bis 90 $\mu$g, am stärksten bevorzugt von 20 $\mu$g bis 80 $\mu$g in 100 $\mu$l Injektionsvolumen betragen. Besonders bevorzugt beträgt die Menge an mRNA 60 $\mu$g in 100 $\mu$l Injektionsvolumen.

Erfindungsgemäße Verwendungen der mRNA und des RL-Injektionpuffers bzw. RL-Injektionspuffers mit Laktat oder der RNA-Injektionslösung sind die Verwendung zur Behandlung und/oder Prophylaxe bzw. zur Herstellung eines Arzneimittels zur Behandlung und/oder Prophylaxe von Krebs- bzw. Tumorerkrankungen, beispielsweise Melanom, wie malignem Melanom, wie malignem Melanom, Hautmelanom, Carzinom, wie Coloncarzinom, Lungencarcinom, wie kleinzelligem Lungencarzinom, Adenocarcinom, Prostatacarcinom, Speiseröhrencarcinom, Brustcarcinom, Nierencarcinom, Sarcom, Myelom, Leukämie, insbesondere AML (akute myeloische Leukämie), Gliom (Glioma), Lymphomen, und Blastomen, Allergien, Autoimmunerkrankungen, wie Multiple Sklerose, viralen und/oder bakteriellen Infektionen.

Auch umfasst die vorliegende Erfindung die Verwendung sowohl der mRNA und des RL-Injektionpuffers bzw. RL-Injektionspuffers mit Laktat als auch der RNA-Injektionslösung u.a. zur Gentherapie und zur Vakzinierung, bspw. zur anti-viralen oder zur Tumor-Vakzinierung, bzw. zur Prävention der vorstehend genannten Erkrankungen.

Eine "Gentherapie" im Sinne der vorliegenden Erfindung bedeutet vor allem, eine fehlende Funktion des Körpers oder der Zelle durch das Einbringen eines funktionierenden Gens in die kranken Zellen wiederherzustellen oder eine störende Funktion durch entsprechende genetische Information zu hemmen. Beispielsweise kann bei einem fehlenden oder nur in geringen Mengen exprimierten Tumorsuppressorgen, z.B. p53, dieses in Form seiner mRNA in die Zelle eingeschleust, in die DNA insertiert und so das ursprünglich defizient exprimierte Protein wieder in physiologisch relevanten Mengen erzeugt werden. Beispielhafte Tumorsuppressorgene im Sinne der vorliegenden Erfindung sind z.B. p53 TP53, RB1, APC, WT1, NF1, NF2, VHL, BRCA1, BRCA2, DCC, MEN 1, MEN 2, PTCH, p57/KIP2, MSH2, MLH1, FMS1, FMS2, MET, p16/INK4a/CDKN2, CDK4, RET, EXT1, EXT2, EXT3, PTEN/MMAC1, ATM, BLM, XPB, XPD, XPA, XPG, FACC, FACA, SMAD4/DPC4, p14$^{Art}$(p19$^{Art}$), DPC4, E-CAD, LKB1/STK1, TSC2, PMS1, PMS2, MSH6, TGF-$\beta$ Typ II R, BAX, $\alpha$-CAT, MADR2/SMAD2, CDX2, MKK4, PP2R1B, MCC, etc..

Eine Vakzinierung im Sinne der Erfindung bedeutet die Einbringung von genetischer Information in Form von mRNA in einen Organismus, insbesondere in eine/mehrere Zelle/Zellen bzw. Gewebe dieses Organismus. Die so verabreichte mRNA wird in dem Organismus das Zielmolekül (z.B. Peptid, Polypeptid, Protein) translatiert, d.h. das von der mRNA kodierte Zielmolekül wird exprimiert und löst eine Immunantwort aus. Es ist bekannt, dass Antigen-präsentierende Zellen (APC) eine obligatorische Schlüsselrolle während des Auslösens einer Immunantwort spielen, da sie den einzigen Zelltyp darstellen, der bei seiner Aktivierung alle Signale auslöst, die für die Initiation der Proliferation von Antigen-spezifischen Immunzellen erforderlich sind. Eine Vakzinierung im Sinne der vorliegenden Erfindung kann beispielsweise erfolgen, indem mRNA verwendet wird, die für ein Antigen kodiert, wobei es sich um Tumorantigene im Falle einer Tumorvakzinierung oder um Fremdantigene im Falle einer Vakzine gegen Fremderreger handeln kann. Beispiele von Tumorantigenen gemäß der vorliegenden Erfindung sind z.B. T-Zell-definierte "Tumorantigene wie bspw. *"Cancer/Testis"-Antigene, z.B.* MAGE, RAGE, NY-ESO-1, *Differenzierungsantigene, z.B.* MART-1/Melan-A, Tyrosinase, gp100, PSA, CD20, *Antigene Epitope mutierter Gene, z.B:* CDK4, Caspase-8 *oder onkofötale Antigene, z.B.* CEA, AF. Andere Tumorantigene sind beispielsweise bei T-Zell und B-Zell-Lymphomen auftretende Tumorantigene CD5 und CAMPATH-1 (CDw52), bei Nicht-Hodgkin'schen B-Zell Lymphomen auftretendes CD20, die bei festen Tumoren, insbesondere bei epithelialen Tumoren (Brust, Darm und Lunge) auftretenden Tumorantigene CEA (carcinoembryogenes Antigen), Mucin, CA-125, sowie FAP-a, Tenascin, und Metalloproteinase, die zusätzlich bei glioblastomen Tumoren auftreten. Weitere Tumorantigene sind bspw. die bei Lunge, Brust, Kopf und Nacken sowie T- und B-Zell-Tumoren auftretenden Tumorantigene EGF (epidermaler Wachstumsfaktor), p185HER2 und der IL-2-Rezeptor, oder das Tumorantigen SV40, etc..

Es kann auch eine mRNA verwendet werden, die für mehrere solcher Antigene kodiert. Dadurch wird erreicht, dass ein Melanom, Carcinom, AML bzw. Gliom effektiv bekämpft werden kann, da eine Kombination aus verschiedenen für den jeweiligen Tumor spezifischen Antigene ein extrem breites Wirkspektrum aufweist. Die erfindungsgemäß verwendete mRNA kann weiterhin für ein immunogenes Protein kodieren. Ein solches immunogenes Protein kann die Reaktivierung einer Immunantwort vermitteln. Einer solchen Reaktivierung liegt die Erkenntnis zugrunde, dass nahezu jeder Organismus sogenannte "Gedächtnis-Immunantworten" gegen gewisse Fremd-Moleküle, z.B. Proteine, insbesondere virale Proteine, Antigene, besitzt. Das bedeutet, dass ein Organismus bereits zu einem früheren Zeitpunkt mit einem solchen Fremd-Molekül infiziert worden ist, und dass durch diese Infektion bereits eine Immunantwort gegen dieses Fremd-Molekül, z.B. ein virales Protein, ausgelöst wurde, die dem Immunsystem im "Gedächtnis" bleibt, d.h. die es speichert. Bei einer erneuten Infektion mit dem gleichen Fremd-Molekül wird diese Immunantwort reaktiviert. Erfindungsgemäß kann eine solche Reaktivierung der Immunantwort durch die Vakzinierung mit einer mRNA erfolgen, die mindestens einen für mindestens ein immunogenes Protein kodierenden Bereich enthält. Bevorzugt ist eine mRNA, die sowohl für ein oder mehrere Antigene als auch für ein oder mehrere immunogene Protein kodiert.

Immunogene Proteine im Sinne der Erfindung sind vorzugsweise Strukturproteine von Viren, insbesondere Matrixproteine, Capsidproteine und Oberflächenproteine der Lipidmembran. Weitere Beispiele für solche viralen Proteine sind Proteine von Adenoviren, Rhinoviren, Corona-Viren, Retroviren. Besonders bevorzugt ist hierbei das Hepatitis B Oberflächen-Antigen ("Hepatits B Surface Antigen", nachfolgend als "HBS-Antigen" bezeichnet) und Influenza-Matrixproteine, insbesondere das Influenza Matrix-M1-Protein.

Beschrieben (nicht beansprucht) wird weiterhin die Verwendung von RNA und des oben beschriebenen RL-Injektionpuffers bzw. RL-Injektionspuffers mit Laktat, sowie des oben beschriebenen RL-Injektionpuffers bzw. RL-Injektionspuffers mit Laktat, oder der oben beschriebenen RNA-Injektionslösung, zur Steigerung des RNA-Transfers und/oder der RNA-Translation von RNA bei "in vitro"-Verfahren, bspw. für Genexpressionsanalysen oder für in-vitro-Screening Verfahren, z.B. via HTS (High Throughput Screening).

Erfindungsgemäß erfolgt die Verwendung einer RNA-Injektionslösung in einem Verfahren zur Steigerung des mRNA-Transfers und/oder der mRNA-Translation von RNA in einem Wirtsorganismus, zur Behandlung und/oder Prophylaxe von Krebs- bzw. Tumorerkrankungen, beispielsweise Melanom, wie malignem Melanom, wie malignem Melanom, Hautmelanom, Carzinom, wie Coloncarzinom, Lungencarcinom, wie kleinzelligem Lungencarzinom, Adenocarcinom, Prostatacarcinom, Speiseröhrencarcinom, Brustcarcinom, Nierencarcinom, Sarcom, Myelom, Leukämie, insbesondere AML (akute myeloische Leukämie), Gliom (Glioma), Lymphomen, und Blastomen, Allergien, Autoimmunerkrankungen, wie Multiple Sklerose, viralen und/oder bakteriellen Infektionen sowie zur Gentherapie und/oder Vakzinierung, ggf. zur anti-viralen Vakzinierung, oder zur Prävention der vorstehend genannten Erkrankungen. Das Verfahren umfasst folgende Schritte:

a.) Herstellen einer RNA-Injektionslösung der vorliegenden Erfindung, und
b.) Verabreichen der RNA-Injektionslösung aus Schritt a.) an einen Wirtsorganismus.

Die Herstellung der RNA-Injektionslösung aus Schritt a. kann wie oben beschrieben erfolgen, d.h. nach jedem beliebigen Verfahren aus dem Stand der Technik, vorzugsweise durch Verdünnen der mRNA in dem RL-Injektionspuffer bzw. RL-Injektionspuffer mit Laktat. Die jeweiligen Konzentrationsverhältnisse sind auch hier abhängig von den oben beschriebenen Voraussetzungen (z.B., Wirtsorganismus, insbesondere Säugetier, dem die RNA-Injektionslösung injiziert wird, Gesundheitszustand, Alter etc. des Wirtsorganismus usw.) zu wählen. Die Verabreichung der RNA-Injektionslösung kann beispielsweise über eine Injektionsspritze (z.B. Sub-Q, Becton Dickinson, Heidelberg, Deutschland) erfolgen. Erfindungsgemäß wird RNA-Injektionslösung intradermal, intraepithelial, intraperitoneal oder intranodal (z.B. in den Lymphknoten) verabreicht.

Bei einem Wirtsorganismus des erfindungsgemäßen Verfahrens handelt es sich vorzugsweise um ein Säugetier, ausgewählt aus der Gruppe bestehend aus Maus, Ratte, Schwein, Rind, Pferd, Hund, Katze, Affe und insbesondere Mensch.

Die hergestellte Injektionslösung kann in einem nicht beanspruchten Aspekt zur in vitro Transfektion von Zellen mit RNA, insbesondere mRNA, verwendet werden. Diese in vitro Transfektion kann für den Laborgebrauch geeignet sein oder Bestandteil einer ex vivo Gentherapie sein, d.h. einer Entnahme von Zellen eines Patienten, einer ex vivo Transfektion von in einer Injektionslösung enthaltenen RNA und anschließender Retransplantation in einen Patienten. Die Transfektion kann mit Hilfe eines Elektroporationsverfahrens durchgeführt werden, ggf. auch unter Anlegen von Spannungspulsen mit einer Feldstärke von max. 2 bis 10 $kVcm^{-1}$ und von Pulsdauern von 10 bis 200 $\mu$s und einer Stromdichte von mindestens 2 $Acm^{-2}$. Sofern nicht für die Transfektion erforderlich, können allerdings auch längere Pulsdauer im Bereich von 1 bis 100 ms verwendet werden. Sofern die Injektionslösung für Laborzwecke eingesetzt wird, können alle denkbaren Laborzellinien auf diese Weise mit RNA transfiziert werden. Für die ex vivo Gentherapie kommen zahlreiche Zelltypen für eine Transfektion in Betracht, insbesondere primäre humane Blutzellen, pluripotente Vorläufer-Blutzellen, aber auch Fibroblasten, Neuronen, Endothelzellen oder Muskelzellen, wobei diese Aufzählung beispielhaft ist und nicht beschränkend gemeint ist.

Die nachfolgenden Figuren und Beispiele dienen der weiteren Erläuterung und Illustration der vorliegenden Erfindung, ohne diese hierauf zu beschränken.

Figuren

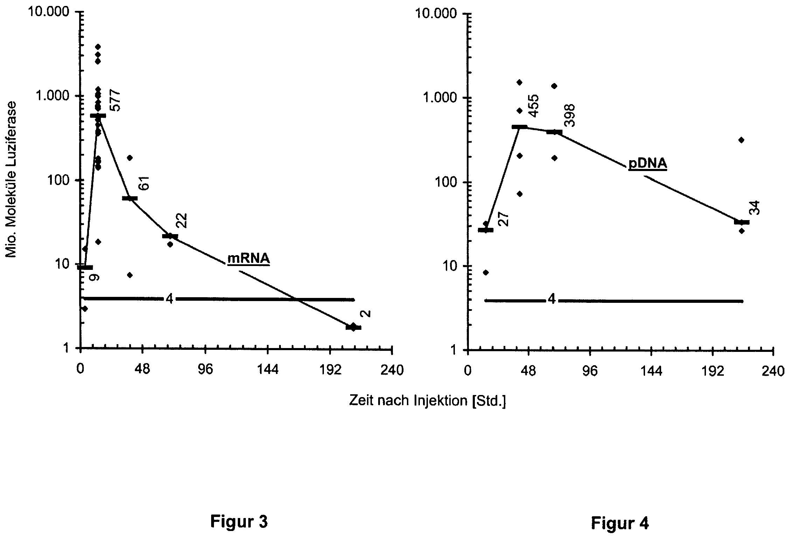

In den in Figuren 1 bis 5 dargestellten Experimenten wurde ein Volumen von 100 $\mu$l des jeweils angegebenen Puffers (Zusammensetzungen der Puffer werden nachfolgend unter Materialien, 1. Injektionspuffer aufgeführt), enthaltend mRNA (Figur 4, pDNA in 100 $\mu$l PBS) die für *Photinus pyralis* Luziferase kodiert, intradermal in die Ohrmuschel von BALB/c Mäusen injiziert[14]. Es wurde die Luziferase-Aktivität eines kompletten Mausohrs analysiert. Diese wird in Million(en) Luziferase-Molekülen angezeigt. Das Detektionslimit wird durch eine dicke Linie, in der eine Zahl angegeben ist, in den Diagrammen angezeigt. Jeder Punkt in den Diagrammen zeigt die Luziferase-Expression eines einzelnen Ohrs an. Kurze Balken mit Zahlen zeigen die Mittelwerte der verschiedenen Gruppen an. Es sind p-Werte für Gruppen angegeben, die signifikant in ihrem Mittelwert (nach Mann-Whitney-Test) abweichen. In Experimenten der Figur 1, 2 und 5 wurden die Ohren 15 Stunden nach der Injektion entfernt. Die gezeigten Daten resultieren aus mindestens drei unabhängigen Experimenten für jede Gruppe.

In **Figur 1** wird ein Vergleich verschiedener Injektionspuffer für mRNA dargestellt: Phosphat-gepufferte Salzlösung (PBS) und HEPES-gepufferte Salzlösung (HBS) und RL-Injektionspuffer mit Laktat (RL). Als Negativkontrolle diente lacZ mRNA. Es wurde erfindungsgemäß gezeigt, dass in RL-Injektionspuffer mit Laktat verdünnte mRNA eine signifikant höhere ($p < 0{,}001$) Expression von Luziferase ergab als mRNA, die in HBS oder PBS verdünnt wurde (Figur 1A).

In **Figur 2** wird der Einfluss des Fehlens von Calcium (-CaCl), Kalium (-KCL) bzw. Natrium Laktat (-NaLa) in dem RL-Injektionspuffer (mit Laktat bzw. ohne Laktat sowie mit bzw. ohne Calcium oder Kalium) auf die Effizienz der Aufnahme der mRNA dargestellt. Der Hauptunterschied zwischen PBS und HBS gegenüber RL-Injektionspuffer bzw. RL-Injektionspuffer mit Laktat (mit und ohne Calcium) besteht in der Abwesenheit von Laktat und Calcium (in HBS bzw. PBS). Daher wurden Untersuchungen durchgeführt, in denen der Transfer und die Translation von Luziferase kodierender mRNA unter Verwendung von einerseits vollständigem RL-Injektionspuffer (RL-Injektionspuffer mit Laktat) und andererseits Formulierungen von RL-Injektionspuffer ohne Calcium bzw. ohne Kalium bzw. ohne Laktat verglichen wurde. Diese Untersuchungen zeigten, dass das Fehlen von Laktat eine Luziferase-Expression ergab, die mit der Expression unter Verwendung von komplettem RL-Injektionspuffer (RL-Injektionspuffer mit Laktat) vergleichbar ist. Im Gegensatz dazu senkte die Abwesenheit von Calcium im RL-Injektionspuffer bzw. RL-Injektionspuffer mit Laktat die Effizienz des RNA-Transfers signifikant ($p = 0{,}004$) auf ein Level, das mit dem von PBS und HBS vergleichbar war.

In **Figuren 3 und 4** wird die Kinetik der mRNA-Translation direkt *in vivo* dargestellt. Es wurden parallele Kinetik Experimente mit RNA in RL mit Laktat und mit DNA in Standardpuffer PBS durchgeführt und gegenübergestellt. Die Translation von mRNA (in RL-Injektionspuffer mit Laktat) **(Figur 3)** oder pDNA (in PBS) **(Figur 4)** für zehn Tage nach der Injektion wurde aufgezeichnet und wird in den Diagrammen dargestellt. In beiden Versuchsdurchführungen (RNA und DNA) wurde die Luziferase-Aktivität in lebenden Mäusen aufgezeichnet. Es werden die Ergebnisse eines repräsentativen Ohres gezeigt. Die Expression von Luziferase, die nach der Injektion von (Luziferase kodierender) mRNA in RL-Injektionspuffer mit Laktat detektiert wurde, erreichte sehr früh (17 Stunden) ihren Höhepunkt und war nach neun Tagen nicht mehr detektierbar **(Figur 3)**. Im Gegensatz dazu ergab die Injektion von (Luziferase kodierender) pDNA in PBS eine spätere Proteinexpression, die nach drei Tagen nach der Injektion ihren Höhepunkt erreichte und für mehr als neun Tage anhielt **(Figur 4)**. Diese Ergebnisse bestätigen erneut nicht nur die Effizienz des erfindungsgemäßen RL-Injektionspuffers mit Laktat, sondern auch, dass RNA als Vehikel für eine transiente Genexpression in Wirtsorganismen, insbesondere Säugetieren, weitaus geeigneter ist als DNA. RNA wird einerseits schneller und zum anderen transient exprimiert, wodurch die gewünschte Genexpression zeitiger und zeitlich begrenzt, und damit gezielter und differenzierter, ausgelöst werden kann. Mit diesen Untersuchungen konnte sowohl der gesteigerte, erfolgreiche RNA-Transfer als auch die effektive nachgeschaltete Translation nachgewiesen werden.

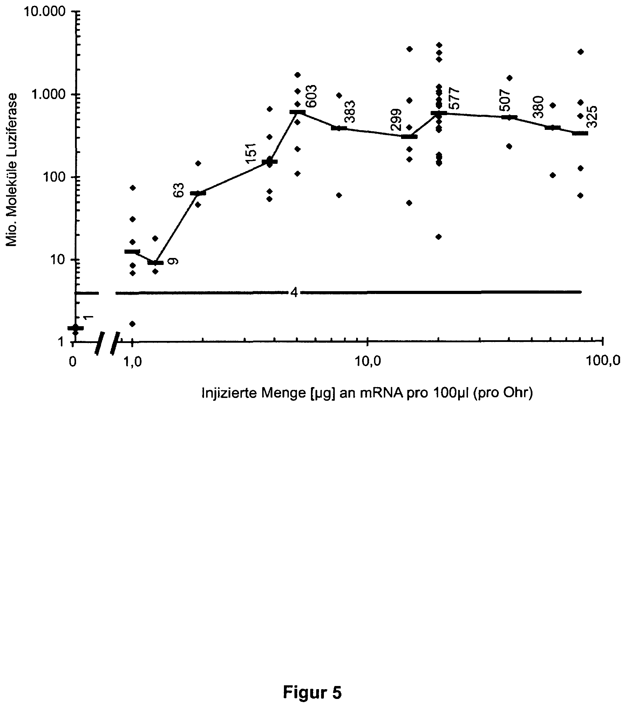

In **Figur 5** wird die Auswirkung unterschiedlicher Mengen von mRNA auf die Luziferase-Expression dargestellt. Diese Experimente wurden insbesondere durchgeführt, um die Dosierung der zu transferierenden RNA in dem RL-Injektionspuffer mit Laktat, insbesondere für klinische Anwendungen, hinsichtlich Menge und Dauer genauer festzulegen. Hierzu wurden mehreren Mäusen ansteigende Mengen an RNA injiziert. Es wurde ein Anstieg der Luziferase-Expression mit ansteigenden Mengen an mRNA bis zu 5 $\mu$g (in 100 $\mu$l Injektionsvolumen) festgestellt. Höhere Dosierungen als 5 $\mu$g führten nicht zu einer weiteren Verbesserung der Expression. In entsprechenden Experimenten beim Menschen (nicht dargestellt) wurde eine Menge von 120 $\mu$g mRNA verwendet, die bis zu 200 $\mu$g gesteigert wurde und zu einer verbesserten Expression führte. Die Menge von 200 $\mu$g mRNA beim Menschen im Vergleich zu 5 $\mu$g bei der Maus lässt sich u.a. aus der Größe der Injektionsstelle ableiten, die beim Menschen ungefähr 40 mal so groß ist. Die humanen Experimente wurden mit gesunden Freiwilligen durchgeführt, nach Aufklärung über Hintergründe und mögliche Konsequenzen der Untersuchungen und nach Einwilligung.

Zur zeitlichen Dosierung ist anzumerken, dass die Translation von mRNA transient erfolgt (wie in **Figur 3** gezeigt, erreicht sie nach 12 Stunden ihren Höhepunkt und ist nach neun Tagen nicht mehr detektierbar) und konsequent reguliert wird. Für eine anhaltende, gleichmäßige Translation des Fremdmoleküls (Protein) ist daher eine Wiederholungsinjektion ungefähr jeden Tag, alle zwei oder alle drei Tage geeignet (abhängig von Faktoren, wie beispielsweise dem Fremdmolekül oder dem Organismus, dem die mRNA injiziert wird).

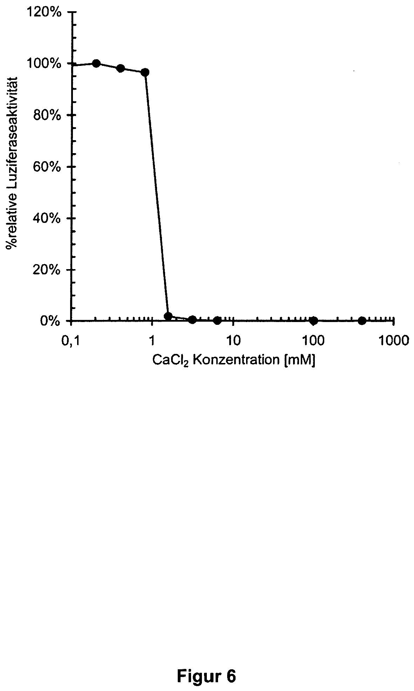

**Figur 6** stellt erneut den Einfluss von CaCl$_2$ auf die Luziferase-Aktivität dar. Es wurde hierzu eine Reihenverdünnung von CaCl$_2$ in Luziferase-Lysispuffer (die Endkonzentrationen sind in dem Diagramm angegeben) angelegt und zu allen Proben die gleiche definierte Menge an rekombinantem Luziferase-Protein hinzugefügt (Endkonzentration ca 4,7 pM). Die Mischungen wurden mit einem Luminometer auf ihre Lichtemission hin untersucht (nach Zugabe von ATP und Luziferin). Anschliessend wurde der Einfluss der CaCl$_2$-Konzentration auf die Luziferase-Aktivität nach folgender Formel berechnet:

$$\% \text{ relative Luziferase-Aktivität (RLA)} = (\text{RLA der Probe mit definierter CaCl}_2\text{-Konzentration} - \text{RLA des reinen Lysispuffers}) / (\text{RLA der Probe ohne CaCl}_2 - \text{RLA des reinen Lysispuffers}) \times 100\%.$$

Die Anwesenheit von Ca$^{2+}$-Ionen bei höherer Konzentration (ab ca. 2 mM) erhöhte die enzymatische Aktivität von Luziferase nicht.

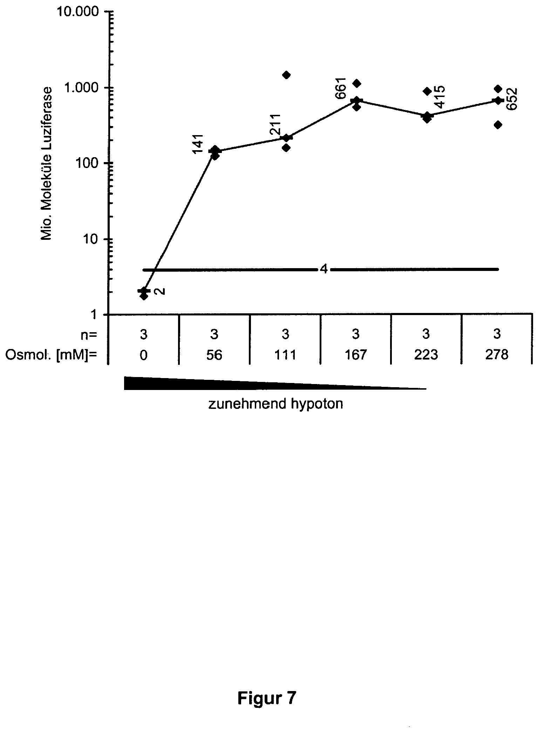

**Figur 7** stellt erneut den Einfluss der CaCl$_2$-Konzentration auf den mRNA-Transfer *in vivo* dar. Es wurden verschiedene Konzentrationen RL-Injektiospuffer mit Laktat verwendet, um RNA-Injektionslösungen (100 μl) mit dergleichen Menge *Photinus pyralis* Luziferase kodierender mRNA (20 μg), aber mit verschiedenen Osmolaritäten (osmol.), bereitzustellen. Die RNA-Injektionslösungen wurden in die Ohrmuschel von BALB/c-Mäusen injiziert. Nach 15 Stunden wurden die Mäuse getötet und Lysate der Ohren angefertigt. Es wird die berechnete Gesamtmenge produzierter Luziferase-Moleküle pro Ohr, der Mittelwert der verschiedenen Gruppen (Balken mit Nummer), die Größe jeder Gruppe (n) und das Detektionslimit des Tests (dicke Linie mit Nummer) angezeigt. Als Ergebnis ist festzuhalten, dass ein effizienter Transfer und damit eine effiziente nachfolgende Translation von mRNA *in vivo* ein Minimum an Ionenkonzentration von 170 mOsm erfordert.

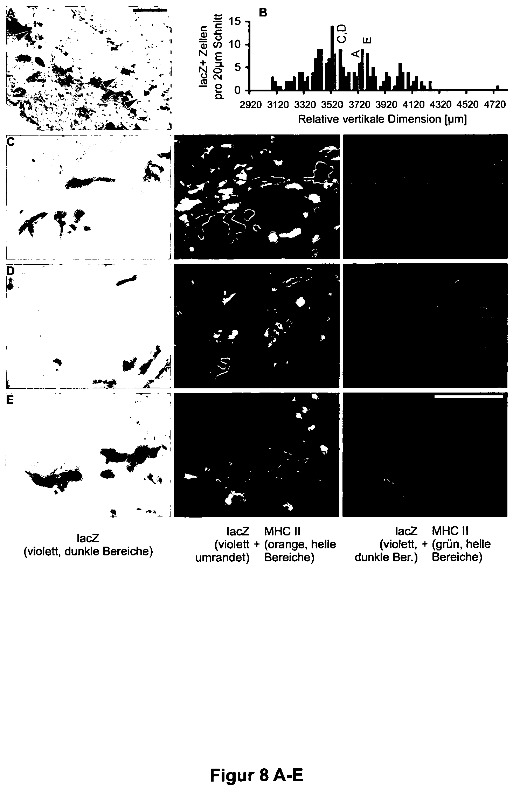

**Figuren 8A-E** stellen die Charakterisierung von Zellen dar, die die zugeführte mRNA *in vivo* exprimieren. Es wurden 20 μg *Eschericha coli* β-Galactosidase kodierende mRNA, verdünnt in einem Gesamtvolumen einer 100 μl RL-Injektionspuffer mit Laktat enthaltenden RNA-Injektionslösung, injiziert. 14 Stunden nach der Injektion wurden die Mäuse getötet, die Ohren entfernt und transversale Kryoabschnitte hergestellt. Die in **Figur 8A** und

**8C bis 8E** dargestellten Abschnitte sind farblich gekennzeichnet.

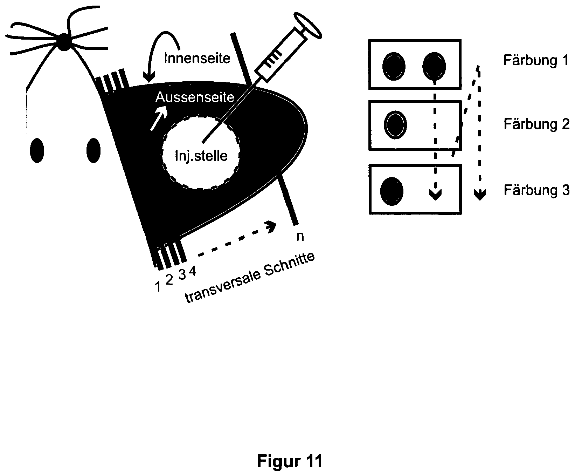

Weiterhin wurde eine gerichtete Genexpression von RNA in RL-Injektionspuffer (mit oder ohne Laktat) untersucht. Hierzu wurde definiert, welche Zelltypen die exogene, in RL-Injektionspuffer (mit oder ohne Laktat) transferierte, RNA aufnehmen und translatieren (siehe auch Beispiel 5, **Figuren 8 A-E,** sowie analog Figuren 11, 12, 16 und 17). Daran anschliessend wurde analysiert, wie im Rahmen einer erfindungsgemäßen mRNA-basierten Vakzinierung eine Immunantwort durch die Translation exogener, in RL-Injektionspuffer (mit oder ohne Laktat) transferierter, RNA in definierten Zielzellen des Immunsystems ausgelöst werden kann.

In Experimenten, dargestellt in **Figur 8A,** wurde jederfünfte Einzelabschnitt mitX-gal-enthaltender Lösung angefärbt. In aufeinanderfolgenden 20 μm dicken Kryoabschnitten wurden bis zu 10 β-Galactosidase positive Zellen detektiert. Das Gebiet der Expression, d.h., β-Galactosidase positive Zellen (durch Pfeile angezeigt), umfasste ein bis zwei Millimeter in Längsrichtung und sagitaler Richtung des Ohrs und war in einer schmalen Schicht zwischen der Epidermis und dem Knorpel der Ohrmuschel lokalisiert.

Erfindungsgemäß wurde weiterhin untersucht, ob APCs ein fremdes Antigen durch direkte Aufnahme und Selbst-Translation der transferierten mRNA erfassen oder durch die Aufnahme des Translationsproduktes der transferierten RNA von anderen Zellen (sog. "cross presentation"). Aufgrund der Lokalisation der Zellen, ihrer Form sowie ihres MHC Klasse II Phänotyps konnte darauf geschlossen werden, dass Zellen, die exogene nackte mRNA an der Injektionsstelle aufnehmen und exprimieren, hauptsächlich Muskelzellen und/oder Fibroblasten sind (Figur 8A). Die Ergebnisse stimmen mit der oben erwähnten "cross presentation" von Antigenen, die von anderen Zellen translatiert wurden, überein. Ein solcher Vorgang würde ebenfalls die Bildung von Antikörpern gegen die Proteine, die durch Nukleinsäuren-Vakzine kodiert werden, erklären. Es konnte damit erfindungsgemäß erstmals festgestellt werden, dass die Auslösung der Immunantwort demnach über ein sog. "cross priming" erfolgt, indem Muskelzellen oder Dermiszellen (Fibroblasten) die Transfer-RNA aufnehmen und exprimieren, und dass die APCs durch diese Zellen aktiviert werden.

Das Histogramm in **Figur 8B** zeigt die Anzahl der β-Galactosidase positiven Zellen in aufeinanderfolgenden Abschnitten. Jeder Balken stellt einen Abschnitt dar.

In Experimenten der **Figuren 8C bis 8E** wurde jeder fünfte Einzelabschnitt gefärbt, und zwar für MHC Klasse II-Expression (detektiert durch Alexa 546-Immunfluoreszenzfärbung, grün) und β-Galactosidase-Expression (detektiert durch Magenta-gal-Färbung, violett). Die Abbildungen in der linken Spalte zeigen eine Magenta-gal-Färbung -alleine- mit β-Galactosidase positiven Zellen in violett = (tief)dunkle Bereiche. In der mittleren und rechten Spalte wird die Überlagerung einer Magenta-gal-Färbung (dargestellt durch Regionen von Interesse in der mittleren und in der rechten Spalte) und MHC Klasse II-Färbung -alleine- (orange = helle Bereiche in der mittleren Spalte) (grün = helle Bereiche in der rechten Spalte) gezeigt. Wie zu sehen ist, erscheinen die meisten β-Galactosidase positiven Zellen eindeutig als MHC Klasse II negativ.

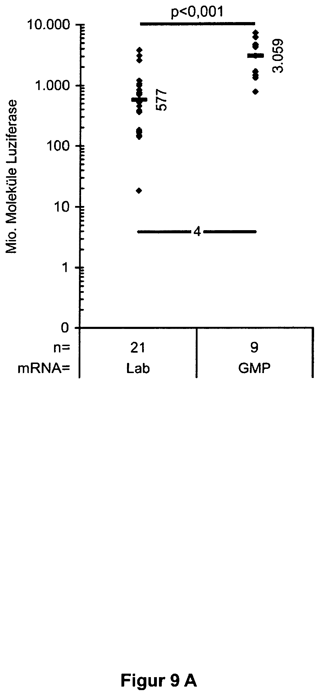

**Figuren 9A-B** zeigen den *in vivo* Transfer von nackter mRNA bei der Maus und beim Menschen. mRNA, für Luziferase kodierend, wurde hergestellt und in RL-Injektionspuffer enthaltender RNA-Injektionslösung aufgelöst. Das Detektionslimit wird durch eine dicke Linie mit einer Zahl in den Diagrammen angezeigt.

In **Figur 9A** gezeigten Experimenten, wurde eine Gesamtmenge von 100 $\mu$l, enthaltend 20 $\mu$g mRNA, in die Ohrmuschel von Mäusen injiziert. 14 Stunden nach der Injektion wurden die Mäuse getötet, die Zellen der Ohren lysiert und das Lysat auf die Luziferase-Expression hin untersucht. Die Anzahl der Luziferasemoleküle pro Ohr wurden berechnet (als Standard wurde rekombinante Luziferase verwendet). Die Daten stammen von mindestens drei unabhängigen Experimenten für jede Gruppe.

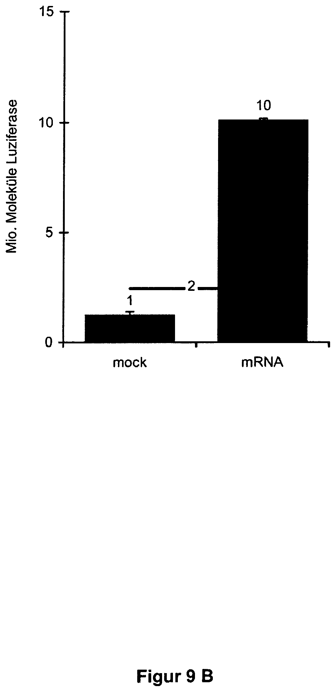

In den in **Figur 9B** gezeigten Experimenten, wurde die gleiche mRNA (120 $\mu$g) in einem Gesamtvolumen von 200 $\mu$l in humaner Haut (in das Bein eines Freiwilligen) injiziert. 16 Stunden später wurden unter Lokalanästhesie Biopsien mit einem Durchmesser von 2 mm entnommen (ausgestanzt), und zwar aus der Mitte der Injektionsstelle ("mRNA") und im Abstand von der Injektionsstelle ("mock"). Eine Luziferase-Aktivität konnte lediglich in der Mitte der Injektionsstelle detektiert werden. Es wird eines von zwei unabhängigen Experimenten gezeigt. Mit diesen Ergebnissen konnte der direkte Transfer nackter mRNA *in vivo* in humane Haut nachgewiesen werden. Damit wird erfindungsgemäß eine effiziente gerichtete mRNA-basierende Vakzinierung bei Menschen ermöglicht.

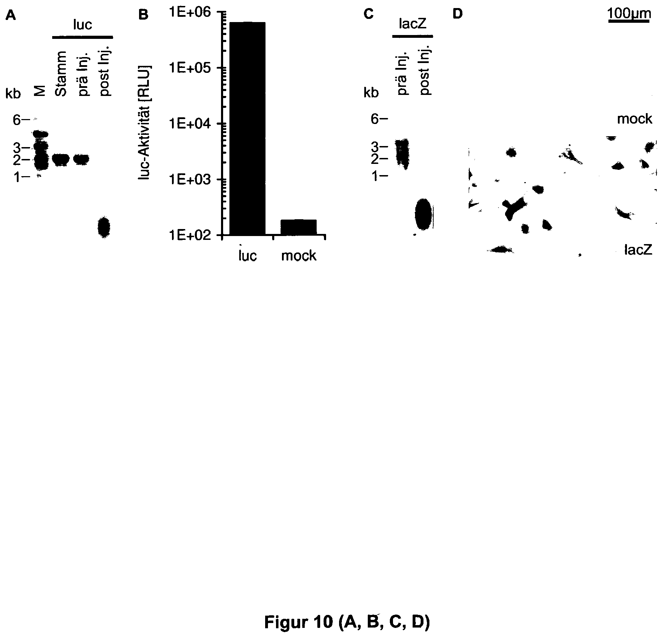

**Figuren 10 A-D** zeigen die Integrität und Translationskapazität der injizierten mRNA in RL-Injektionspuffer mit Laktat. Die Integrität wurde mit Formaldehyd-Agarose-Gelelektrophorese (1,2% w/v) überprüft. Hierfür wurde 1 $\mu$g mRNA, kodierend entweder für *Photinus pyralis* Luziferase (luc, 1.9 kB, **Figur 10A**) oder für *Eschericha coli* β-Galactosidase (lacZ, 3,5 kB, **Figur 10C**) aufgetrennt. Es ist kein Unterschied in der Integrität der mRNA (vor der Injektion) vor der Verdünnung im jeweiligen Injektionspuffer (Stammlösung) und nach der Verdünnung im jeweiligen Injektionspuffer detektiert worden. Im Gegensatz dazu ist eine sichtbare, vollständige Degradierung der mRNA (nach der Injektion) festzustellen, wenn die Reste der RNA-Injektionslösung aus der Injektionsspritze gesammelt werden.

Offensichtlich sind diese Reste durch den Kontakt der Injektionsspritze mit dem Maus- bzw. Menschgewebe mit Ribonukleasen kontaminiert worden.

Die Translationskapazität der injizierten mRNA wurde durch Elektroporation von BHK21-Zellen mit 10 $\mu$g mRNA überprüft. Als Kontrolle wurden entweder 10 $\mu$g irrelevanter mRNA oder keine mRNA verwendet (mock). Nachfolgend wurden die Zellen entweder lysiert und mit einem Luminometer auf ihre Luziferase-Aktivität hin untersucht **(Figur 10B)** oder mit X-gal gefärbt und mit einem Lichtmikroskop auf ihre Luziferase-Aktivität hin untersucht **(Figur 10D)**.

**Figur 11** stellt die Identifikation des mRNA-Transfers auf zellulärer Ebene dar. Das Schema zeigt die Aufsicht auf eine Maus. In dem Schema ist die äußere (dorsale) Seite direkt für den Betrachter sichtbar. Der Maus wurde mRNA in RL-Injektionspuffer mit Laktat in die Ohrmuschel injiziert. Es wurden aufeinanderfolgende transversale Abschnitte von dem Ohr (1, 2, 3, 4) angefertigt. Die Abschnitte wurden in verschiedenen Sätzen (1, 2, 3, 4) gesammelt, luftgetrocknet und bis zu den verschiedenen Färbungsvorgängen bei -20°C aufbewahrt.

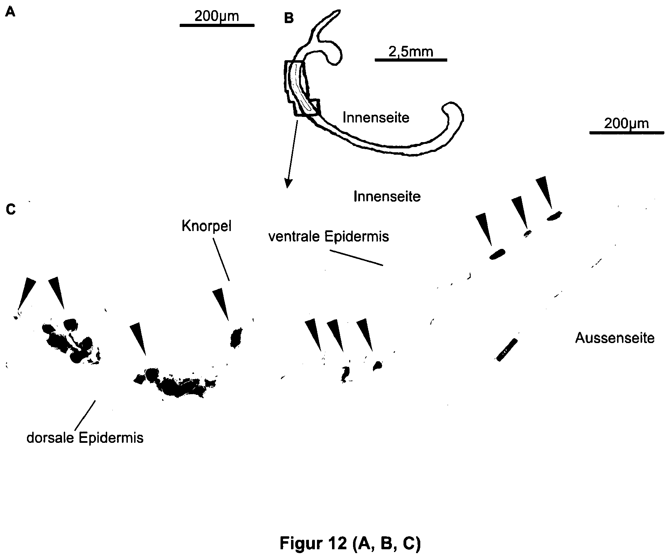

**Figuren 12A-C** zeigen den Transfer der mRNA auf zellulärer Ebene. Es wurden 5 $\mu$g *Eschericha coli* β-Galactosidase kodierende mRNA in RL-Injektionspuffer mit Laktat in ein Mausohr injiziert. 15 Stunden nach der Injektion wurde das Ohr in TissueTek O.C.T Medium eingebettet und 60 $\mu$m dicke Kryoabschnitte angefertigt. Die Abschnitte wurden über Nacht mit X-gal gefärbt. **Figur 12A** stellt Kryoabschnitte eines mRNA-Transfer negativen Ohres dar. Es sind keine lacZ positiven Zellen detektierbar. **Figur 12B** zeigt einen Überblick. **Figur 12C** zeigt eine Detailansicht eines mRNA-Transfer positiven Ohres. lacZ positive Zellen erscheinen dunkelblau und werden durch Pfeile angezeigt.

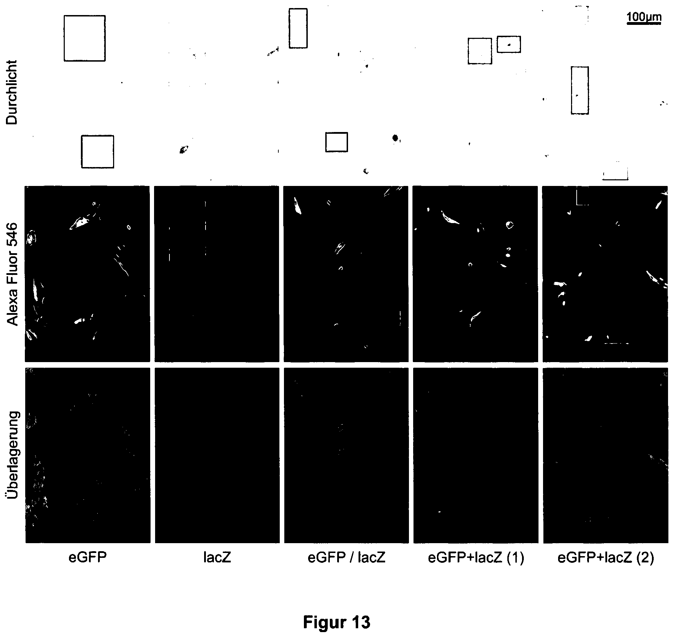

**Figur 13** zeigt die Kompatibilität des Alexa Fluor 546-Signals mit der Farbe der Magenta-gal positiven Zellen. Um zu bestimmen, ob die Detektion von Alexa Fluor 546 in Magenta-gal positiven Zellen möglich ist, wurden BHK-Zellen mit Kombinationen von eGFP mRNA (eGFP = enhanced green fluorescence protein) oder lacZ mRNA transfiziert. Folgende Kombinationen transfizierter Zellen wurden analysiert:

- Einzeltransfektionen mit nur eGFP mRNA oder lacZ mRNA eine Mischung aus solchen einzeln transfizierten Zellen (eGFP/lacZ) und
- doppelt transfizierte Zellen (eGFP+lacZ).

Die Zellen wurden mit einem anti-eGFP-Antikörper mit Alexa Fluor 546-Detektion und nachfolgend mit Magenta-gal gefärbt. Es wurden Magenta-gal gefärbte positive Zellen (die lacZ exprimieren) mit Breitfeldlichtmikroskopie detektiert (obere Reihe) und Alexa Fluor 546 gefärbte positive Zellen (die eGFP exprimieren) mit Fluoreszenzmikroskopie detektiert (mittlere Reihe). Es wurden Überlagerungen von beiden Ergebnissen vorgenommen (untere Reihe), um genaue Resultate über die Lokalisation der Zellen zueinander zu erhalten, obwohl das Alexa 546-Signal in dieser Darstellung die Abbildung des Lichtmikroskops verdeckt. Zwar konnte nicht ausgeschlossen werden, dass die direkte Aufnahme und Selbst-Translation der zugeführten mRNA in den APCs stattfindet und ausreichend ist, um eine Immunantwort auszulösen. Es könnten in einigen APCs Vorgänge einer geringen oder unvollständigen nicht detektierbaren Translation, erfolgt sein und (im Fall einer unvollständigen Translation) die Prozessierung und Präsentation des fremden Antigens bewirkt haben.

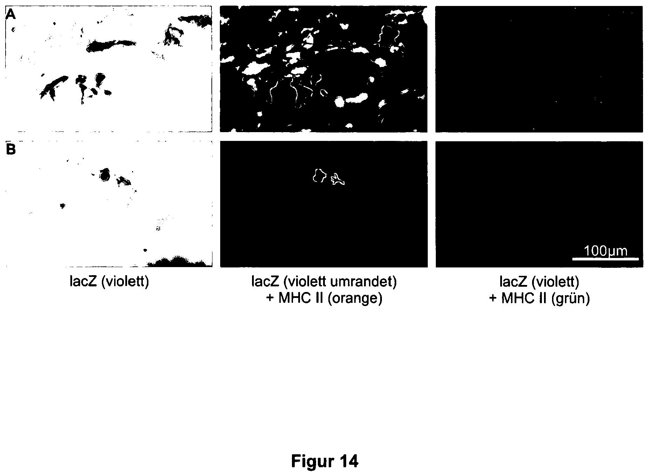

**Figuren 14 A-B** zeigen die Spezifität von MHC Klasse II-Färbungen von Kryoabschnitten. Es wurden 20 μg *Eschericha coli* β-Galactosidase kodierende mRNA in einem Gesamtvolumen von 100μl RL-Injektionspuffer mit Laktat injiziert. 14 Stunden nach der Injektion wurden die Mäuse getötet und die Ohren entfernt. Es wurden transversale Kryoabschnitte hergestellt. Die Kryoabschnitte wurden zuerst mit einem anti-MHC Klasse II-Antikörper **(Figur 14A)** oder dem entsprechenden Isotyp-Kontroll-Antikörper **(Figur 14B)** gefärbt und mit immunfluoreszenter Färbung mit Alexa 546 detektiert. Dann wurden die Kryoabschnitte mit Magenta-gal (für β-Galactosidase-Expression) gefärbt. Die Abbildungen zeigen Magenta-gal-Färbungen (linke Säule), MHC Klasse II-Färbungen (mittlere Säule, Position von lacZ positiven Zellen sind durch Umrandungen angezeigt) und eine Überlagerung aus beiden Färbungen (rechte Säule, lacZ positive Zellen sind durch Umrandungen angezeigt, MHC Klasse II positive Zellen stellen die hellen Bereiche in der Abbildung dar).

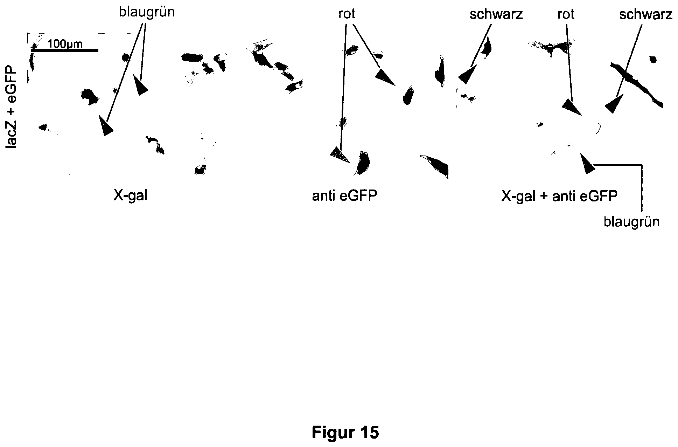

**Figur 15** stellt die Kompatibilität von Zellen dar, die X-gal-Farbstoff und AEC-Farbstoff positiv sind. Um zu bestimmen, ob das X-gal Präzipitat mit der Detektion von AEC positiven Zellen kompatibel ist, wurden BHK-Zellen mit eGFP mRNA und lacZ mRNA co-transfiziert. Die Zellen wurden mit einer anti-eGFP-Immunfärbung mit AEC (rot: positive Zellen, exprimieren eGFP), mit einer X-gal-Lösung (blau-grün: positive Zellen, exprimieren lacZ) oder mit einer Kombination aus AEC und X-gal gefärbt. Die gefärbten Zellen wurden mit Breitlichtmikroskopie analysiert. Doppelt positive Zellen erscheinen schwarz (schwarze Pfeile). Die Unterscheidung von einzeln gefärbten positiven Zellen (grüne und rote Pfeile) wird schwierig, wenn die Einzelfärbung stark ausfällt und daher eher schwarz erscheint.

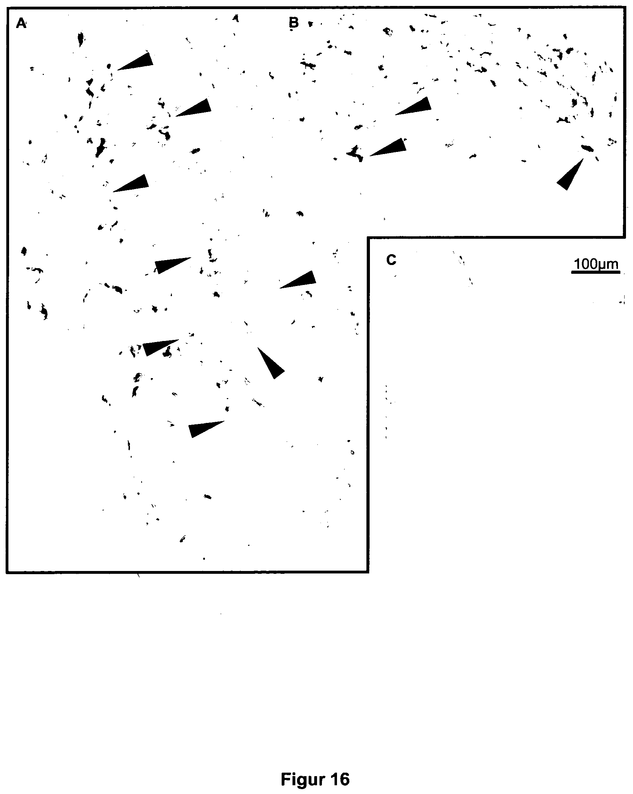

**Figuren 16 A-B** zeigen die Co-Lokalisation von MHC Klasse II positiven und mRNA-Transfer positiven Zellen. Es wurden 20 μg β-Galactosidase kodierende mRNA in einem Gesamtvolumen von 100 μl RL-Injektionspuffer mit Laktat injiziert. 14 Stunden nach der Injektion wurden die Mäuse getötet und die Ohren entfernt. Es wurden transversale Kryoabschnitte angefertigt, die zuerst mit einem anti-MHC Klasse II-Antikörper **(Figur 16A+B)** oder dem entsprechenden Isotyp-Kontroll-Antikörper **(Figur 16C)** (mit mit Alexa 546 Färbung detektiert), dann mit X-gal (für β-Galactosidase-Expression) gefärbt wurden. Zellen, die für den mRNA-Transfer positiv sind, erscheinen grün-blau, Zellen, die für MHC Klasse II positiv sind, erscheinen rot und doppelt positive Zellen erscheinen schwarz. mRNA-Transfer positive Zellen werden unabhängig von der MHC Klasse II-Expression durch einen Pfeil angezeigt.

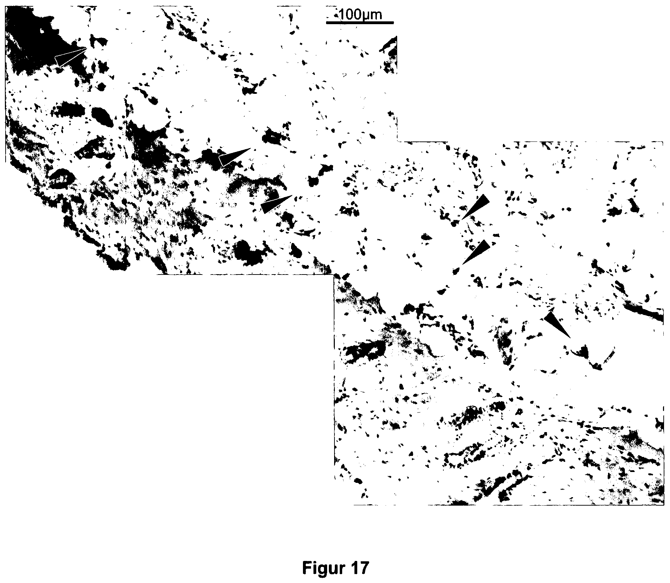

**Figur 17** stellt den mRNA-Transfer und die Morphologie der Ohrmuschel dar. Es wurden 20 μg ß-Galactosidase kodierende mRNA in einem Gesamtvolumen von 100 μl RL-Injektionspuffer mit Laktat injiziert. 14 Stunden nach der Injektion wurden die Mäuse getötet und die Ohren entfernt. Es wurden transversale Kryoabschnitte hergestellt, die zuerst mit X-gal (für β-Galactosidase-Expression), dann mit Hematoxylin und Eosin gefärbt wurden. Zellen, die für den mRNA-Transfer positiv sind, werden durch Pfeile angezeigt und sind nahe der Parenchymzellen-Schicht angesiedelt.

**Beispiele**

**Materialien**

**1. Injektionspuffer**

Es wurden folgende Puffer verwendet:

- 2x Phosphat-gepufferte Salzlösung (PBS)
  (PBS 274 mM Natriumchlorid, 5,4 mM Kaliumchlorid, 20 mM Dinatriumhydrogenphosphat, 4 mM Kaliumdihydrogenphosphat, pH 7,3 bei 20,8 °C),
- 2x HEPES-gepufferte Salzlösung (HBS)
  (HBS: 300 mM Natriumchlorid, 20 mM Hepes, pH 7,4 bei 20,8 °C) und
- 1 x RL-Injektionspuffer (ohne Laktat)

  (82,2 mM Natriumchlorid, 4,3 mM Kaliumchlorid, 1,44 mM Calciumchlorid, falls keine andere Zusammensetzung und Konzentration angegeben wurde). 1 x RL-Injektionspuffer mit Laktat
  (102,7 mM Natriumchlorid, 5,4 mM Kaliumchlorid, 1,8 mM Calciumchlorid, 20 mM Natriumlaktat, falls keine andere Zusammensetzung und Konzentration angegeben wurde).

- 1 x RL-Injektionspuffer mit Laktat, ohne Natriumchlorid
  (4,3 mM Kaliumchlorid, 1,44 mM Calciumchlorid, 22,4 mM Natriumlaktat, falls keine andere Zusammensetzung und Konzentration angegeben wurde).
- 1 x RL-Injektionspuffer mit Laktat, ohne Kaliumchlorid (82,2 mM Natriumchlorid, 1,44 mM Calciumchlorid, 22,4 mM Natriumlaktat, falls keine andere Zusammensetzung und Konzentration angegeben wurde).

- 1 x RL-Injektionspuffer mit Laktat, ohne Calciumchlorid
  (82,2 mM Natriumchlorid, 4,3 mM Kaliumchlorid, 22,4 mM Natriumlaktat, falls keine andere Zusammensetzung und Konzentration angegeben wurde).

Bei Verwendung von 2x PBS und 2x HBS wurden alle Komponenten in Wasser aufgelöst und der pH eingestellt. Dann wurde Diethylpyrocarbonat (DEPC, Sigma, Schnelldorf, Deutschland) hinzugefügt, bis zu einer Konzentration von 0,1 % (v/v). Die Puffer wurden für über eine Stunde bei 37°C inkubiert. Anschließend wurden die Puffer autoklaviert.

1 x RL-Injektionspuffer mit Laktat wurde selbst hergestellt aus einer 20x Stammlösung der vier verschiedenen Salze (Natriumchlorid, Kaliumchlorid, Calciumchlorid und Natriumlaktat). Ebenso wurde der 1 x RL-Injektionspuffer hergestellt aus einer 20x Stammlösung der drei verschiedenen Salze (Natriumchlorid, Kaliumchlorid und Calciumchlorid). Bei weiteren Experimenten wurde Natriumchlorid bzw. Kaliumchlorid bzw. Calciumchlorid weggelassen, ohne die niedrigere Osmolarität zu kompensieren. Auch diese RL-Injektionspuffer mit Laktat, ohne NaCl, KCl oder $CaCl_2$ wurden aus einer 20x Stammlösung hergestellt. Mit Ausnahme der Natriumlaktat-Racemat-Lösung (Fluka, Schnelldorf, Deutschland), wurde jede dieser Komponenten mit DEPC behandelt und autoklaviert, wie für 2x PBS und 2x HBS beschrieben.

Sämtliche Puffer und Pufferkomponenten wurden auf Ribonuklease-Aktivität hin untersucht, indem 1 $\mu$g mRNA in 1 x Puffer für mehr als zwei Stunden bei 37°C inkubiert wurde. Bei der Analyse der mRNA mittels Formaldehyd-Agarose-Gelelektrophorese wurden Puffer verwendet, bei denen keine Degradation beobachtet wurde.

### 2. Mäuse

Alle Tierexperimente wurden in Übereinstimmung mit den institutionellen und nationalen Richtlinien durchgeführt. Es wurden weibliche BALB/c Mäuse mit einem Alter von 8 - 15 Wochen von Charles River (Sulzfeld, Deutschland) bezogen.

Vor der intradermalen Injektion wurden die Mäuse anästhesiert und die Ohrmuschel mit Isopropanol behandelt. Um die mRNA-Aufnahme und -Translation zu analysieren, wurden die Mäuse nach einer bestimmten Zeit getötet, die Ohren wurden entfernt und mit einer Rasierklinge rasiert, um störende Haare zu entfernen.

### 3. Mensch

Die humanen Experimente wurden mit gesunden männlichen Freiwilligen durchgeführt, die über die Hintergründe und möglichen Konsequenzen der Untersuchungen aufgeklärt wurden, und die ihre Einwilligung erteilten.

### Beispiel 1: Herstellung der Nukleinsäuren

mRNA

Es wurde "capped" (gekappte) mRNA mittels *in vitro* "run-off" Transkription mit T7 RNA Polymerase (T7-Opti mRNA kits, CureVac, Tübingen, Deutschland) hergestellt.

Die kodierende Sequenz dieser mRNA (entweder *Eschericha coli* $\beta$-Galactosidase [lacZ] kloniert von Acc. U02445, oder *Photinus pyralis* Luziferase [luc], kloniert von Acc. U47295) wurde an ihren 3'-Enden durch eine alpha-Globin nicht-translatierte Region und einen künstlichen poly A (n=70)-Schwanz flankiert. Für die Mausexperimente wurde die mRNA mit Phenol/Chloroform/Isoamylalkohol extrahiert und mit Lithiumchlorid präzipitiert. Die mRNA wurde nachfolgend in Wasser resuspendiert und die Ausbeute spektrometrisch bei 260 nm bestimmt. Schließlich wurde die mRNA mit Ammoniumacetat präzipitiert und steril in Wasser resuspendiert.

pDNA

Es wurde Endotoxin-freie pCMV-luc DNA mit dem EndoFree Plasmid Maxi Kit (Qiagen, Hilden, Deutschland) hergestellt. Die pDNA wurde mit Ammoniumacetat präzipitiert und schließlich steril in Wasser resuspendiert. Das pCMV-luc Plasmid wurde durch Insertion eines Xba 1- (blunted mit Klenow Fragment) Hind III-Fragments von pGL3 (Acc. U47295) in das Nsi I- (blunted mit Klenow Fragment) Hind III-verdaute Plasmid von pCMV-HB-S (Acc. A44171) modifiziert. Das Reportergen der pDNA stand unter der Kontrolle des CMV-Promoters.

Stammlösungen

Es wurden Stammlösungen hergestellt, indem die mRNA bzw. DNA in sterilem Wasser verdünnt wurde und die Konzentration und Reinheit spektrometrisch (bei 260, 280 and 320 nm) bestimmt wurde.

Qualitätskontrolle

Für alle Nukleinsäureproben wurde die Konzentration spektrometrisch bestimmt und die Integrität mittels Formaldehyd-Agarose-Gelelektrophorese (mRNA) oder Restriktionsverdauung und TBE-Agarose-Gelelektrophorese (DNA) überprüft (Figur 10). Zusätzlich zu der Integrität wurde die Translationskapazität von allen Nukleinsäureproben durch Elektroporation von BHK21-Zellen analysiert. Hierfür wurden 1-3 Mio. Zellen in 200 $\mu$l PBS mit 10 $\mu$g Nukleinsäure bei 300 V und 150 $\mu$F in 0,4 cm Küvetten elektroporiert. Die transfizierten Zellen wurden 8-24 Stunden nach der Elektroporation durch ein geeignetes Detektionsverfahren (X-gal-Färbung oder Lumineszenz-Detektion) auf die Proteinexpression hin analysiert (Figur 10). Für *in vivo* Experimente wurden nur Nukleinsäureproben injiziert, die eine Proteinexpression in BHK21-Zellen und eine passende Integrität in der Gelelektrophorese zeigten.

## Beispiel 2: Zubereitung der RNA-Injektionslösungen

Für HBS und PBS wurde die mRNA in 1x konzentriertem Puffer verdünnt. Für RL-Injektionspuffer mit bzw. ohne Laktat und die einzelnen Variationen von diesem (Abwesenheit von einem der Ionen $Ca^{2+}$, $K^+$, $Na^+$) (Zusammensetzungen und Konzentrationen siehe Materialien, 1. Injektionspuffer) wurde die mRNA in 0,8 x konzentriertem Puffer verdünnt. Sofern nichts anderes angegeben wird, wurden 20 $\mu$g mRNA in 100 $\mu$l Injektionspuffer pro Mausohr verwendet. Um Sekundärstrukturen in der mRNA zu entfernen, wurden die RNA-Injektionslösungen für 5 Minuten auf 80°C erhitzt. Danach wurden die Lösungen auf Eis für weitere 5 Minuten gestellt. Schließlich wurde die RNA-Injektionslösung in Sub-Q (Becton Dickinson, Heidelberg, Deutschland)-Injektionsspritzen aufgezogen. Für jede Injektion wurden separate Injektionsspritzen verwendet. Plasmid-DNA (pDNA) wurde in 1 x konzentriertem PBS verdünnt.

## Beispiel 3: Detektion der Luziferase-Aktivität *ex vivo*

Um die Luziferase-Expression *ex vivo* zu detektieren, wurden Gewebelysate angefertigt. Hierfür wurde das Gewebe unter flüssigem Stickstoff mit Mörser und Stößel zerkleinert und die restlichen "Klumpen" mit 800 $\mu$l Lysispuffer (25 mM Tris HCl, 2 mM EDTA, 10% (w/v) Glycerin, 1 % (w/v) Triton X-100 plus frisch hinzugegebenem 2 mM DTT und 1 mM PMSF) homogenisiert. Der Überstand des Homogenats wurde nach Zentrifugation (10 min, 13.000 rpm, 4°C) in einer Minizentrifuge gewonnen. Aliquots von 110 $\mu$l dieses Lysats wurden bei -80°C aufbewahrt.

Um die Luziferase-Aktivität zu messen, wurden Aliquots auf Eis aufgetaut und die Lichtemission von 50 $\mu$l Lysat für 15 Sekunden mit einem Luminometer (LB 9507, Berthold, Bad Wildbad, Deutschland) gemessen. Das Luminometer fügte automatisch vor der Messung 300 $\mu$l Puffer A (25 mM Glycyl-glycin, 15 mM Magnesiumsulphat, 5 mM frisch zugefügtes ATP, pH 7,8) und 100 $\mu$l Puffer B (250 $\mu$M Luziferin in Wasser) zu dem Lysat hinzu.

Zur Standardisierung wurden in allen Messungen Reihenverdünnungen von rekombinantem Luziferase-Protein (QuantiLum®, Promega, Madison, USA) verwendet. Anhand dieses Standards wurde die Menge an Luziferase-Molekülen für jede einzelne Messung berechnet. Für jedes Lysat wurde die Luziferase-Aktivität an zwei verschiedenen Tagen doppelt gemessen und der Mittelwert der Luziferase-Aktivität berechnet. Der Variationskoeffizient (n = 4) für die Menge an Luziferase-Molekülen lag unter 10% für alle Lysate mit Luziferase-Aktivität oberhalb des Detektionslimits. Dieses Detektionslimit (angezeigt durch eine dicke Linie mit Nummer in allen Diagrammen) wurde anhand des Mittelwerts der Messungen mit nur Lysispuffer plus dreimal der Standardabweichung dieser Werte (n = 80) berechnet.

## Beispiel 4: *In vivo* Biolumineszenz-Detektion

Um eine Luziferase-Injektion in lebenden Tieren zu detektieren, wurden Mäuse zu einem bestimmten Zeitpunkt nach der Nukleinsäure-Injektion anästhesiert. Die Mäuse wurden in drei unterschiedliche Gruppen eingeteilt: Gruppe I von Mäusen wurde in das linke Ohr 100 $\mu$l RL-Injektionspuffer, in das rechte Ohr 20 $\mu$g Luziferase-kodierende mRNA in 100 $\mu$l RL-Injektionspuffer, in injiziert. Gruppe II wurde in linkes und rechtes Ohr jeweils 20 $\mu$g Luziferase-kodierende mRNA in 100 $\mu$l RL-Injektionspuffer, injiziert. Gruppe III von Mäusen wurde in das rechte Ohr 100 $\mu$l RL-Injektionspuffer, in das linke Ohr 20 $\mu$g Luziferase-kodierende mRNA in 100 $\mu$l RL-Injektionspuffer, injiziert. Dann wurde den Mäusen i.p. 200 $\mu$l 20 mg/ml Luziferin (Synchem, Kassel, Deutschland) in PBS (steril filtriert) injiziert. 5 Minuten nach der Luziferin-Injektion wurde die Lichtemission der Mäuse für einen Zeitraum von 20 Minuten gesammelt. Hierzu wurden die Mäuse auf einer vorgewärmten Platte (37°C) in einer verdunkelten Box positioniert (Gruppe I links, Gruppe II Mitte, Gruppe III rechts). Die Box war ausgestattet mit einer Aequoria Macroscopic Imaging Kamera (Hamamatsu, Japan), platziert. Die Lichtemission wurde in einem Falschfarbenbild dargestellt, welches einem Graustufenbild der Maus überlagert ist. der Mäuse bei normalem Licht dargestellt. Das gleiche Experiment wurde analog unter Verwenden von 20 $\mu$g Luziferase-kodierender mRNA in RL-Injektionspuffer mit Laktat bzw. RL-Injektionspuffer mit Laktat, ohne Natriumchlorid, RL-Injektionspuffer mit Laktat, ohne Kaliumchlorid bzw. RL-Injektionspuffer mit Laktat, ohne Calciumchlorid durchgeführt.

**Beispiel 5: β-Galactosidase-Aktivität und -Histologie**

Rasierte Mausohren wurden zerlegt, in Medium, enthaltend Tissue-Tek® O.C.T™ Verbindung (Sakura, Zoeterwuode, Niederlande), eingebettet und bei -80°C gelagert. Aus diesen Blöcken wurden 20 aufeinanderfolgende 20 μm dicke transversale Kryoabschnitte in 5 Sätzen (Figur 11) auf SuperFrost® plus Objektträgern (Langenbrinck, Emmendingen, Deutschland) so platziert, dass der vertikale Abstand zwischen zwei Abschnitten eines Satzes ungefähr 100 μm betrug. Dann wurden die Abschnitte luftgetrocknet und bis zu ihrer Färbung bei -20°C gelagert. Für ein erstes Screening, in welchem Gebiet die transferierte mRNA (kodierend für *Eschericha coli* β-Galactosidase) aufgenommen und translatiert worden ist, wurde 1 Satz Abschnitte mit X-gal gefärbt. Hierfür wurden die Objektträger Raumtemperatur ausgesetzt und mit einem ImmEdge™ Pen (Vektor, Burlingame, USA) umzeichnet. Dann wurden die Abschnitte für 15 Minuten mit 2% Formalin in PBS fixiert. Danach wurden die Objektträger 3x für 2 Minuten PBS gewaschen und dann übernacht bei 37°C in einer Feuchtekammer mit X-gal-Färbelösung (1 mg/ml frisch zugegebenem X-gal, 5 mM Kaliumferricyanid, 5 mM Kaliumferrocyanid, 1 mM Magnesiumchlorid, 15 mM Natriumchlorid, 60 mM Dinatriumhydrogenphosphat, 40 mM Natriumdihydrogenphosphat) gefärbt. Die Färbung wurde abgeschlossen, indem die Objektträger 2x für 2 Minuten gewaschen wurden und mit Hydro-Matrix®- (Micro-Tech-Lab, Graz, Österreich, zweifach verdünnt in Wasser) Medium behandelt.

Um Informationen über die Gewebsmorphologie zu erhalten, wurde für einen anderen Satz Abschnitte die X-gal-Färbung mit einer Hematoxylineosin(HE)-Färbung kombiniert. Hierfür wurden die Abschnitte nach der X-gal-Färbung 3x für 2 Minuten in PBS und zusätzlich 5 Minuten in bidestilliertem Wasser gewaschen, bevor eine 2 Sekunden dauernde Färbung mit Mayers Hämalaun (Merck, Darmstadt, Deutschland) durchgeführt wurde. Die Färbung wurde für 10 min unter fließendem Leitungswasser entwickelt, bevor für 10 min die Gegenfärbung mit 0,1 % Eosin Y (Sigma, Schnelldorf, Deutschland) in Wasser erfolgte. Die Färbung wurde durch kurzes Waschen in bidestilliertem Wasser gestoppt, gefolgt von der Dehydration mit ansteigenden Alkoholkonzentrationen (2 Minuten 80% Ethanol, 2 Minuten 95% Ethanol, 2 Minuten 100% Ethanol, 5 Minuten 100% Xylol). Schließlich wurden die getrockneten Abschnitte mit Roti®-Histokitt (Roth, Karlsruhe, Deutschland) Medium behandelt.

Um festzustellen, ob es sich bei den mit der mRNA transfizierten Zielzellen um Antigen-präsentierende Zellen handelt, wurde eine Doppelfärbung für MHC Klasse II Moleküle (exprimiert durch APC) und mRNA-Transfer (bezüglich β-Galactosidase-Expression) durchgeführt. Es wurde sowohl eine immunhistochemische als auch eine immunfluoreszente Detektion der MHC-Klasse II Moleküle durchgeführt. Für beide Protokolle wurden die Abschnitte zwischen allen Schritten 3x für 2 Minuten mit PBS gewaschen. Für die immunhistochemische Vorgehensweise wurden die Abschnitte mit 1% (w/v) Formalin (Fluka) in PBS fixiert. Dann wurden die Lipide durch Inkubation für 30 Sekunden in reinem Aceton entfernt. Unmittelbar danach wurde für 30 Minuten bei Raumtemperatur mit 4% Ziegenblut (Vektor Laboratories Inc., Burlingame, CA) und 50 μg/ml Avidin D (Vektor Laboratories Inc., Burlingame, CA) in PBS geblockt. Die verbleibenden Biotin-Bindungsseiten wurden mit 50 μg/ml Biotin (AppliChem, Darmstadt, Deutschland) geblockt und gleichzeitig für MHC Klasse II Moleküle mit dem monoklonalen Antikörper 2G9 (Becton Dickinson, Heidelberg, Deutschland) oder dem geeigneten Isotyp-Kontroll-Antikörper (Ratten-IgG 2a, R35-95, Becton Dickinson, Heidelberg, Deutschland), jeweils auf 1 μg/ml (alle in PBS) verdünnt, gefärbt. Danach wurden die Abschnitte für 30 Minuten bei Raumtemperatur inkubiert mit Biotinyliertem Ziege / anti-Ratte-IgG (3 μg/ml) Vektor und 2% Mausserum (CCPro, Neustadt, Deutschland) in PBS inkubiert. Nachfolgend wurde ABC-Komplex (1 : 100 von Reagenz A und B in PBS, (Vektor Laboratories Inc., Burlingame, CA) für 30 Minuten bei Raumtemperatur hinzugefügt. Die MHC Klasse II Färbung wurde vervollständigt durch Detektion mit frisch präparierter und 0,45 μm filtrierter 3-Amino-9-Ethylcarbazol- (AEC, Sigma) Substratlösung (0,5 mg/ml AEC, 0,015% Hydrogenperoxid, 50 mM Natriumacetat, pH 5,5). Die Substratreaktion wurde durch zweimaliges Waschen für 5 Minuten mit Wasser und dreimaligem Waschen für 5 Minuten mit PBS gestoppt. Nachfolgend wurde eine X-gal-Färbung durchgeführt, wie oben beschrieben.

Für die immunfluoreszente Detektion wurde ein ähnliches Färbungsprotokoll verwendet. Auf den Aceton-Schritt folgend wurden die Abschnitte für 50 Minuten bei Raumtemperatur in Blocking-Puffer (1% Rinderserum Albumin in PBS) geblockt. Die Abschnitte wurden dann mit primären Antikörpern (2G9 oder Isotyp-Kontroll-Antikörper), verdünnt auf 1 μg/ml in Blocking-Puffer, für 40 Minuten inkubiert. Danach erfolgte eine Inkubation für 40 Minuten bei Raumtemperatur mit Alexa Fluor 546 Ziege/anti-Ratte-IgG (1:400; Molecular Probes, Leiden, Niederlande) in Blocking-Puffer. Schließlich wurde eine Magenta-gal-Färbung durchgeführt. Hierfür wurde X-gal in der Färbungslösung durch 0,1 mg/ml Magenta-gal (Peqlab, Erlangen, Deutschland) ersetzt.

Die Abschnitte wurden mit einem Zeiss (Oberkochen, Deutschland) Axioplan 2 Microscope, ausgestattet mit einer Axiocam HRc Kamera und der Axiovision 4.0 Software, analysiert. Farben und Kontrast in den Fotographien wurden in linearer Weise eingestellt.

**Beispiel 6: RNA-Transfer und -Translation beim Menschen**

Dieses Experiment wurde mit gesunden männlichen Freiwilligen durchgeführt. Die Injektionsstellen wurden rasiert, desinfiziert und mit RNaseZap (Ambion, Austin, USA) Lösung behandelt. Dann wurden in einem Ansatz 120 $\mu$g mRNA in 0,8x RL-Injektionspuffer in einem Gesamtvolumen von 200 $\mu$l injiziert. In weiteren analogen Ansätzen wurde anstelle von RL-Injektionspuffer:

- RL-Injektionspuffer mit Laktat, ohne Natriumchlorid,
- RL-Injektionspuffer mit Laktat, ohne Kaliumchlorid und
- RL-Injektionspuffer mit Laktat, ohne Calciumchlorid injiziert.

15 Stunden nach der Injektion wurden Biopsien mit einem Durchmesser von 4 mm Durchmesser unter lokaler Anästhesie entnommen (ausgestanzt). Die Biopsien wurden in flüssigem Stickstoff schockgefroren und wie beschrieben zubereitet (Beispiel 3). Die zermahlenen Biopsien wurden in 600 $\mu$l Lysispuffer resuspendiert.

Statistische Bewertung

Es wurden die Mittelwerte von zwei verschiedenen Gruppen mit dem sog. "nonparametric Mann-Whitney rank sum test" verglichen. Ein p-Wert von <0,05 wurde als signifikanter Unterschied angesehen und in den Diagrammen dargestellt.

Beispiel 7: Anmerkungen zu den verschiedenen durchgeführten Färbungsverfahren

Um den Zelltyp zu identifizieren, der die mRNA *in vivo* aufnimmt und exprimiert, wurde ein histologisches Verfahren verwendet, das die Detektion des mRNA-Transfers in Verbindung mit einer Zelltyp-spezifischen Färbung ermöglicht.

Da der mRNA-Transfer nicht mit fluoreszierenden Sonden detektiert werden konnte, wurde ein Verfahren durchgeführt, bei dem *Eschericha coli* $\beta$-Galactosidase-kodierende mRNA in Kombination mit verschiedenen Indigo-Farbstoffen (X-gal oder Magenta-gal) verwendet wurde.

1. Spezielle Merkmale des $\beta$-Galactosidase-Detektionssystems

Um sicherzustellen, dass für das Indigo-Färbungsverfahren alle Zellen in einer Einzelschicht vorlagen, wurden dünne Abschnitte des Mausohrs angefertigt. Unter Berücksichtigung der Morphologie der Ohrmuschel der Mäuse (eine dünne Schicht von ungefähr 0,5-1 mm Dicke) und der Tatsache, dass nur Kryoabschnitte verwendet werden können (ß-Galactosidase wird während einiger Schritte, die erforderlich sind, um Paraffinabschnitte herzustellen, hitzeinaktiviert), und dem Erfordernis von Abschnitten und möglichst zahlreichen Unterabschnitten mit hoher Qualität, erwies sich die Anfertigung dieser Abschnitte als sehr schwierig. Dennoch war es möglich, mehrere Sätze von Abschnitten guter Qualität mit einer Dicke von 20 $\mu$m herzustellen. Es wurden zwei verschiedene Farbstoffe verwendet, um die $\beta$-Galaktosidase-Aktivität zu detektieren. Für X-gal (positive Zellen färbten sich blau-grün) wurden Ergebnisse mit einem besseren Kontrast erzielt als mit Magenta-gal (positive Zellen färbten sich violett). Gleichzeitig waren jedoch unspezifische Hintergrundfärbungen, beispielsweise verursacht durch Haarfolikel, stark bei der X-gal-Färbung zu erkennen. Dennoch war eine klare Unterscheidung von dem mRNA-Transfer möglich. Eine unspezifische Hintergrundfärbung war für Magenta-gal nicht zu erkennen.

2. Erfordernisse für eine kombinierte Indigo- und Immun-Färbung

Die Kombination von mRNA-Transfer-Färbung (Indigo-Farbstoff) und Zellspezifischer-Marker-Färbung (spezifische Antikörper) erforderte mehrere Anpassungen bzgl. des Fixiermittels und der Reihenfolge der kombinierten Färbungen (Indigo-Färbung umfasste eine 14-Stunden lange Inkubation bei 37°C). Die besten Ergebnisse für die Antikörper-Färbung (gegen MHC II Moleküle) wurden erzielt, wenn zuerst eine Aceton-Fixierung und die Antikörper-Färbung durchgeführt wurde. Im Gegensatz dazu wurden die besten Ergebnisse für die $\beta$-Galaktosidase-Aktivität erzielt, wenn zuerst eine Fixierung mit einer Mischung aus Formaldehyd und Glutardialdehyd und die Indigo-Färbung durchgeführt wurde. Unter Berücksichtigung dieser verschiedenen Sachverhalte wurde folgendes Verfahren ausgewählt: Fixierung mit Formaldehyd, jedoch ohne Glutardialdehyd und der Antikörper-Färbung. Glutardialdehyd musste weggelassen werden, da es die Autofluoreszenz von dem Gewebe drastisch erhöht, während es lediglich einen geringen, wenn überhaupt irgendeinen, Effekt auf die Qualität der Indigo-Färbung hatte. Die Fixierung mit Formaldehyd wurde aus mehreren Gründen verwendet:

1. die Morphologie des Gewebes wurde hierdurch besser geschützt als mit Aceton,
2. eine scharfe und starke Indigo-Färbung erfordert eine Fixierung mit Formaldehyd und

3. die Qualität der Anti MHC II Antikörper-Färbung war dennoch mit Formaldehyd akzeptabel.

Es wurde eine kurze Inkubation in reinem Aceton vorgenommen, um Lipide und Fette zu entfernen. Dies ermöglichte eine bessere Qualität (wenige bzw. keine Luftblasen) mit dem verwendeten wasserlöslichen Medium. Schließlich wurden Antikörper-Färbungen von guter Qualität lediglich dann erreicht, wenn diese Färbung zuerst durchgeführt wurde. Die Färbungsreihenfolge (Formaldehyd-Fixierung) hatte lediglich einen geringen Effekt auf die Qualität der Indigo-Färbung.

3. Farbstoff-Kompatibilität in kombinierten Indigo- und Immun-Färbungen

Die Kombination von zwei verschiedenen Färbungen erforderte nicht nur die Kompatibilität der verschiedenen Schritte der beiden Protokollen, sondern auch die Kompatibilität der für die Detektion verwendeten Sonden. Grundsätzlich ist eine Immun-Färbung mit präzipitierenden Farbstoffen (enzymatische Sonde) oder mit fluoreszierenden Farbstoffen (markierte Sonde) möglich. Um die Indigo-Färbung mit einem präzipitierenden Farbstoff zu kombinieren, wurden X-gal und AEC verwendet. Doppelt positive Zellen erschienen in einer solchen Färbung schwarz (Figur 15). Die Unterscheidung von intensiv gefärbten einzelnen positiven Zellen kann hierbei schwierig sein. Daher wurde die Kombination von Magenta-gal mit dem fluoreszierenden Farbstoff Alexa Fluor 546 bevorzugt. Die Verwendung von Magenta-gal anstelle von X-gal wurde aus zwei Gründen bevorzugt:

1. Die Intensität der Magenta-gal-Färbung war durchweg schwächer (sogar, wenn der Farbstoff in gesättigten Mengen hinzu gegeben wurde),
2. die Farbe von Magenta-gal positiven Zellen stimmte besser mit der Emissions-Wellenlänge von dem Alexa Fluor 546 fluoreszierenden Farbstoff überein.

Beide Faktoren minimieren ein Quenchen des Alexa Fluor 546 fluoreszierenden Signals. Diese Farbstoffkombination ermöglichte tatsächlich die Detektion von beiden Signalen (Figur 13), zumindest dann, wenn die Indigo-Färbung nicht zu stark ausfiel (dies war durchweg der Fall für die mRNA-Transfer positiven Zellen in den Abschnitten).

Literaturliste

1. Ulmer J.B., Curr. Opin. DrugDiscov. Devel, 2001, 4:192-197
2. J. A. Wolff et al., Science 247, 1465-1468 (1990).
3. H. L. Robinson, L. A. Hunt, R. G. Webster, Vaccine 11, 957-960 (1993).
4. J. B. Ulmer et al., Science 259, 1745-1749 (1993).
5. J. A. Wolff et al., Science 247, 1465-1468 (1990).
6. D. Boczkowski, S. K. Nair, D. Snyder, E. Gilboa, J.Exp.Med. 184, 465-472 (1996).
7. J. P. Carralot et al., Cell Mol.Life Sci. (2004).
8. R. M. Conry et al., Cancer Res. 55, 1397-1400 (1995).
9. R. D. Granstein, W. Ding, H. Ozawa, J Invest Dermatol 114, 632-636 (2000).
10. I. Hoerr, R. Obst, H. G. Rammensee, G. Jung, Eur. J Immunol. 30, 1-7 (2000).
11. J. Donnelly, K. Berry, J. B. Ulmer, Int J Parasitol. 33, 457-467 (2003).
12. D. M. Klinman et al., Springer Semin.Immunopatho/. 19, 245-256 (1997).
13. J.-P. Carralot et al., CMLS Cell. Mol. Life Sci. 61, 2418-2424 (2004).
14. P. Forg, P. von Hoegen, W. Dalemans, V. Schirrmacher, Gene Ther. 5, 789-797 (1998).

**Patentansprüche**

1. Verwendung von mRNA und einem wässrigen Injektionspuffer, der ein Natriumsalz, ein Calciumsalz und ein Kaliumsalz enthält, zur Herstellung eines Arzneimittels zur Behandlung und/oder Prophylaxe von Krebs- bzw. Tumorerkrankungen, Allergien, Autoimmunerkrankungen, viralen und/oder bakteriellen Infektionen, zur Gentherapie oder zur Vakzinierung, insbesondere anti-viralen Vakzinierung, zur Prävention der vorstehend genannten Erkrankungen, wobei das Arzneimittel eine mRNA-Injektionslösung zur Steigerung des mRNA-Transfers und/oder der mRNA-Translation in einen/einem Wirtsorganismus enthält und die mRNA-Injektionslösung intradermal, intraepithelial, intraperitoneal oder intranodal verabreicht wird.

2. Verwendung nach Anspruch 1, wobei der Injektionspuffer Salze mit den Anionen, ausgewählt aus der Gruppe, bestehend aus Chloriden, Iodiden, Bromiden, Hydroxiden, Carbonaten, Hydrogencarbonaten oder Sulfaten, enthält, insbesondere der Injektionspuffer als Salze NaCl, $CaCl_2$ und KCl enthält.

3. Verwendung nach Anspruch 2, wobei der Injektionspuffer mindestens 50 mM Natriumchlorid (NaCl), mindestens 0,01 mM Calciumchlorid ($CaCl_2$) und mindestens 3 mM Kaliumchlorid (KCl) enthält.

4. Verwendung nach einem der Ansprüche 1 bis 3, wobei der Injektionspuffer 50 mM bis 800 mM, bevorzugt 60 mM bis 500 mM, stärker bevorzugt 70 mM bis 250 mM, insbesondere bevorzugt 60 mM bis 110 mM Natriumchlorid (NaCl); 0,01 mM bis 100 mM, bevorzugt 0,5 mM bis 80 mM, stärker bevorzugt 1,5 mM bis 40 mM Calciumchlorid ($CaCl_2$); und 3 mM bis 500 mM, bevorzugt 4 mM bis 300 mM, stärker bevorzugt 5 mM bis 200 mM Kaliumchlorid (KCl) enthält.

5. Verwendung nach einem der Ansprüche 1 bis 4, wobei der Injektionspuffer ein Puffersystem enthält.

6. Verwendung nach einem der vorangehenden Ansprüche 1 bis 5, wobei die mRNA nackte mRNA, oder mRNA, komplexiert mit einem Polykation ist.

7. Verwendung nach Anspruch 6, wobei die mRNA mit Protamin komplexiert ist.

8. Verwendung nach einem der Ansprüche 6 oder 7, wobei die mRNA mindestens eine natürliche oder nicht natürlich auftretende Modifikation aufweist, wobei die Modifikation insbesondere ausgewählt ist aus der Gruppe, bestehend aus Erhöhung des G/C-Gehalts im codierenden Bereich der mRNA ohne Veränderung der codierten Aminosäuresequenz, Einführung mindestens eines Ribonukleotid-Analogons, Eliminierung von destabilisierenden Sequenzen in der WT-Sequenz oder Erhöhung des A/U-Gehalts im Bereich der Ribosomenbindungsstelle.

9. mRNA-Injektionslösung zur Steigerung des mRNA-Transfers und/oder der mRNA Translation in einen/einem Wirtsorganismus, enthaltend mRNA und einen wässrigen Injektionspuffer, der ein Natriumsalz, ein Calciumsalz und ein Kaliumsalz enthält, zur Verwendung in der Behandlung und/oder Prophylaxe von Krebs- bzw. Tumorerkrankungen, Allergien, Autoimmunerkrankungen, viralen und/oder bakteriellen Infektionen, zur Gentherapie oder zur Vakzinierung, insbesondere zur antiviralen Vakzinierung, zur Prävention der vorstehend genannten Erkrankungen, wobei die mRNA-Injektionslösung intradermal, intraepithelial, intraperitoneal oder intranodal verabreicht wird.

10. mRNA-Injektionslösung zur Verwendung nach Anspruch 9, wobei der Injektionspuffer Salze mit den Anionen, ausgewählt aus der Gruppe, bestehend aus Chloriden, Iodiden, Bromiden, Hydroxiden, Carbonaten, Hydrogencarbonaten oder Sulfaten, enthält, insbesondere der Injektionspuffer als Salze NaCl, $CaCl_2$ und KCl enthält.

11. mRNA-Injektionslösung zur Verwendung nach Anspruch 10, wobei der Injektionspuffer mindestens 50 mM Natriumchlorid (NaCl), mindestens 0,01 mM Calciumchlorid ($CaCl_2$) und mindestens 3 mM Kaliumchlorid (KCl) enthält.

12. mRNA-Injektionslösung zur Verwendung nach einem der Ansprüche 9 bis 11, wobei der Injektionspuffer 50 mM bis 800 mM, bevorzugt 60 mM bis 500 mM, stärker bevorzugt 70 mM bis 250 mM, insbesondere bevorzugt 60 mM bis 110 mM Natriumchlorid (NaCl); 0,01 mM bis 100 mM, bevorzugt 0,5 mM bis 80 mM, stärker bevorzugt 1,5 mM bis 40 mM Calciumchlorid ($CaCl_2$); und 3 mM bis 500 mM, bevorzugt 4 mM bis 300 mM, stärker bevorzugt 5 mM bis 200 mM Kaliumchlorid (KCl) enthält.

13. mRNA-Injektionslösung zur Verwendung nach einem der Ansprüche 9 bis 12, wobei die mRNA nackte mRNA, oder mRNA, komplexiert mit einem Polykation ist.

14. mRNA-Injektionslösung zur Verwendung nach Anspruch 13, wobei die mRNA mit Protamin komplexiert ist.

15. mRNA-Injektionslösung zur Verwendung nach einem der Ansprüche 13 oder 14, wobei die mRNA mindestens eine natürliche oder nicht natürlich auftretende Modifikation aufweist, wobei die Modifikation insbesondere ausgewählt ist aus der Gruppe, bestehend aus Erhöhung des G/C-Gehalts im codierenden Bereich der mRNA ohne Veränderung der codierten Aminosäuresequenz, Einführung mindestens eines Ribonukleotid-Analogons, Eliminierung von destabilisierenden Sequenzen in der WT-Sequenz oder Erhöhung des A/U-Gehalts im Bereich der Ribosomenbindungsstelle.

16. mRNA-Injektionslösung zur Verwendung nach Anspruch 9, wobei die Krebs- bzw. Tumorerkrankung ausgewählt ist aus der Gruppe bestehend aus Melanom, Hautmelanom, Carzinom, wie Coloncarzinom, Lungencarcinom, Adenocarcinom, Prostatacarcinom, Speiseröhrencarcinom, Brustcarcinom, Nierencarcinom, Sarcom, Myelom, Leukämie, insbesondere AML (akute myeloische Leukämie), Gliom (Glioma), Lymphomen, und Blastomen.

## Claims

1. Use of mRNA and an aqueous injection buffer containing a sodium salt, a calcium salt and a potassium salt, for the preparation of a medicament for the treatment and/or prevention of cancer or tumor diseases, allergies, autoimmune diseases, viral and/or bacterial infections, for gene therapy or for vaccination, in particular anti-viral vaccination, for the prevention of the above cited diseases, wherein the medicament contains an mRNA injection solution for increasing the mRNA transfer and/or mRNA translation into/in a host organism, and wherein the mRNA injection solution is administered intradermally, intraepithelially, intraperitoneally or intranodally.

2. Use according to claim 1, wherein the injection buffer contains salts with the anions selected from the group consisting of chlorides, iodides, bromides, hydroxides, carbonates, hydrogen carbonates or sulfates, in particular the injection buffer contains NaCl, $CaCl_2$ and KCl as salts.

3. Use according to claim 2, wherein the injection buffer contains at least 50 mM sodium chloride (NaCl), at least 0.01 mM calcium chloride ($CaCl_2$) and at least 3 mM potassium chloride (KCl).

4. Use according to any one of claims 1 to 3, wherein the injection buffer contains from 50 mM to 800 mM, preferably from 60 mM to 500 mM, more preferably from 70 mM to 250 mM, particularly preferably from 60 mM to 110 mM sodium chloride (NaCl); from 0.01 mM to 100 mM, preferably from 0.5 mM to 80 mM, more preferably from 1.5 mM to 40 mM calcium chloride ($CaCl_2$); and optionally from 3 mM to 500 mM, preferably from 4 mM to 300 mM, more preferably from 5 mM to 200 mM potassium chloride (KCl).

5. Use according to one of claims 1 to 4, wherein the injection buffer contains a buffer system.

6. Use according to any one of the preceding claims 1 to 5, wherein the mRNA is naked mRNA, or mRNA complexed with a polycation.

7. Use according to claim 6, wherein the mRNA is complexed with protamine.

8. Use according to any one of claims 6 or 7, wherein the mRNA has at least one natural or non-naturally occurring modification, wherein the modification is selected in particular from the group consisting of increasing the G/C content in the coding region of the mRNA without changing the coded amino acid sequence, introducing at least one ribonucleotide analogue, eliminating destabilising sequences in the WT sequence or increasing the A/U content in the region of the ribosome binding site.

9. An mRNA injection solution for increasing the mRNA transfer and/or mRNA translation into/in a host organism containing mRNA and an aqueous injection buffer containing a sodium salt, a calcium salt and a potassium salt, for use in the treatment and/or prevention of cancer or tumor diseases, allergies, autoimmune diseases, viral and/or bacterial infections, for gene therapy or for vaccination, in particular anti-viral vaccination, for the prevention of the above cited diseases, wherein the mRNA injection solution is administered intradermally, intraepithelially, intraperitoneally or intranodally.

10. The mRNA injection solution for use according to claim 9, wherein the injection buffer contains salts with the anions selected from the group consisting of chlorides, iodides, bromides, hydroxides, carbonates, hydrogen carbonates or sulfates, wherein in particular the injection buffer contains NaCl, $CaCl_2$ and KCl as salts.

11. The mRNA injection solution for use according to claim 10, wherein the injection buffer contains at least 50 mM sodium chloride (NaCl), at least 0.01 mM calcium chloride ($CaCl_2$) and at least 3 mM potassium chloride (KCl).

12. The mRNA injection solution for use according to any of claims 9 to 11, wherein the injection buffer contains from 50 mM to 800 mM, preferably from 60 mM to 500 mM, more preferably from 70 mM to 250 mM, particularly preferably from 60 mM to 110 mM sodium chloride (NaCl); from 0.01 mM to 100 mM, preferably from 0.5 mM to 80 mM, more preferably from 1.5 mM to 40 mM calcium chloride ($CaCl_2$); and from 3 mM to 500 mM, preferably from 4 mM to 300 mM, more preferably from 5 mM to 200 mM potassium chloride (KCl).

**13.** The mRNA injection solution for use according to any of claims 9 to 12, wherein the mRNA is naked mRNA, or mRNA complexed with a polycation.

**14.** The mRNA injection solution for use according to claim 13, wherein the mRNA is complexed with protamine.

**15.** The mRNA injection solution for use according to any of claims 13 or 14, wherein the mRNA has at least one natural or non-naturally occurring modification, wherein the modification is selected in particular from the group consisting of increasing the G/C content in the coding region of the mRNA without changing the coded amino acid sequence, introducing at least one ribonucleotide analogue, eliminating destabilising sequences in the WT sequence or increasing the A/U content in the region of the ribosome binding site.

**16.** The mRNA injection solution for use according to claim 9, wherein the cancer or tumor disease is selected from the group consisting of melanoma, skin melanoma, carcinoma, such as colon carcinoma, lung carcinoma, adenocarcinoma, prostate carcinoma, esophageal carcinoma, breast carcinoma, kidney carcinoma, sarcoma, myeloma, leukemia, particularly AML (acute myeloid leukemia), glioma (Glioma), lymphomas, and blastomas.

**Revendications**

**1.** Utilisation d'ARNm et d'un tampon aqueux pour injection qui contient un sel de sodium, un sel de calcium et un sel de potassium, pour la préparation d'un médicament pour le traitement et/ou la prophylaxie de cancers ou d'affections tumorales, d'allergies, de maladies auto-immunes, d'infections virales et/ou bactériennes, pour la thérapie génique ou pour la vaccination, en particulier une vaccination antivirale, pour la prophylaxie de maladies précédentes dans laquelle le médicament contient une solution d'ARNm pour injection pour l'augmentation du transfert d'ARNm et/ou de la traduction d'ARNm dans un organisme hôte et la solution d'ARNm pour injection est administrée par voie intradermique, intra-épithéliale, intrapéritonéale ou intranodale.

**2.** Utilisation selon la revendication 1, dans laquelle le tampon pour injection contient des sels avec les anions choisis dans le groupe constitué de chlorures, iodures, bromures, hydroxydes, carbonates, hydrogénocarbonates ou sulfates, en particulier le tampon pour injection contient comme sels du NaCl, du $CaCl_2$ et du KCl.

**3.** Utilisation selon la revendication 2, dans laquelle le tampon pour injection contient au moins 50 mM de chlorure de sodium (NaCl), au moins 0,01 mM de chlorure de calcium ($CaCl_2$) et au moins 3 mM de chlorure de potassium (KCl).

**4.** Utilisation selon l'une des revendications 1 à 3, dans laquelle le tampon pour injection contient 50 mM à 800 mM, de préférence 60 mM à 500 mM, de manière davantage préférée, 70 mM à 250 mM, de manière particulièrement préférée, 60 mM à 110 mM de chlorure de sodium (NaCl) ; 0,01 mM à 100 mM, de préférence 0,5 mM à 80 mM, de manière davantage préférée, 1,5 mM à 40 mM de chlorure de calcium ($CaCl_2$) ; et 3 mM à 500 mM, de préférence 4 mM à 300 mM, de manière davantage préférée, 5 mM à 200 mM de chlorure de potassium (KCl).

**5.** Utilisation selon l'une des revendications 1 à 4, dans laquelle le tampon pour injection contient un système de tampon.

**6.** Utilisation selon l'une des revendications précédentes 1 à 5, dans laquelle l'ARNm est un ARNm nu ou un ARNm complexé à un polycation.

**7.** Utilisation selon la revendication 6, dans laquelle l'ARNm est complexé à une protamine.

**8.** Utilisation selon l'une des revendications 6 ou 7, dans laquelle l'ARNm présente au moins une modification naturelle ou survenant de façon non naturelle, dans laquelle la modification est choisie en particulier dans le groupe constitué d'une élévation de la teneur en G/C dans le domaine codant de l'ARNm sans modification de la séquence d'acides aminés codante, l'introduction d'au moins un analogue de ribonucléotide, l'élimination de séquences déstabilisantes dans la séquence WT ou l'élévation de la teneur en A/U dans le domaine du site de liaison ribosomique.

**9.** Solution d'ARNm pour injection pour l'augmentation du transfert d'ARNm et/ou de la traduction d'ARNm dans un organisme hôte contenant un ARNm et un tampon aqueux pour injection qui contient un sel de sodium, un sel de calcium et un sel de potassium, pour son utilisation dans le traitement et/ou la prophylaxie de cancers ou d'affections tumorales, d'allergies, de maladies auto-immunes, d'infections virales et/ou bactériennes, pour la thérapie génique ou pour la vaccination, en particulier la vaccination antivirale, pour la prophylaxie de maladies précédentes dans laquelle la solution d'ARNm pour injection est administrée par voie intradermique, intraépithéliale, intrapéritonéale ou intranodale.

10. Solution d'ARNm pour injection pour son utilisation selon la revendication 9, dans laquelle le tampon pour injection contient des sels avec les anions choisis dans le groupe constitué de chlorures, iodures, bromures, hydroxydes, carbonates, hydrogénocarbonates ou sulfates, en particulier le tampon pour injection contient comme sels du NaCl, du $CaCl_2$ et du KCl.

11. Solution d'ARNm pour injection pour son utilisation selon la revendication 10, dans laquelle le tampon pour injection contient au moins 50 mM de chlorure de sodium (NaCl), au moins 0,01 mM de chlorure de calcium ($CaCl_2$) et au moins 3 mM de chlorure de potassium (KCl).

12. Solution d'ARNm pour injection pour son utilisation selon l'une des revendications 9 à 11, dans laquelle le tampon pour injection contient 50 mM à 800 mM, de préférence 60 mM à 500 mM, de manière davantage préférée, 70 mM à 250 mM, de manière particulièrement préférée, 60 mM à 110 mM de chlorure de sodium (NaCl) ; 0,01 mM à 100 mM, de préférence 0,5 mM à 80 mM, de manière davantage préférée, 1,5 mM à 40 mM de chlorure de calcium ($CaCl_2$) ; et 3 mM à 500 mM, de préférence 4 mM à 300 mM, de manière davantage préférée, 5 mM à 200 mM de chlorure de potassium (KCl).

13. Solution d'ARNm pour injection pour son utilisation selon l'une des revendications 9 à 12, dans laquelle l'ARNm est un ARNm nu ou un ARNm complexé à un polycation.

14. Solution d'ARNm pour injection pour son utilisation selon la revendication 13, dans laquelle l'ARNm est complexé à une protamine.

15. Solution d'ARNm pour injection pour son utilisation selon l'une des revendications 13 ou 14, dans laquelle l'ARNm présente au moins une modification naturelle ou survenant de façon non naturelle, dans laquelle la modification est choisie en particulier dans le groupe constitué d'une élévation de la teneur en G/C dans le domaine codant de l'ARNm sans modification de la séquence d'acides aminés codante, l'introduction d'au moins un analogue de ribonucléotide, l'élimination de séquences déstabilisantes dans la séquence WT ou l'élévation de la teneur en A/U dans le domaine du site de liaison ribosomique.

16. Solution d'ARNm pour injection pour son utilisation selon la revendication 9, dans laquelle le cancer ou l'affection tumorale est choisi(e) dans le groupe constitué d'un mélanome, d'un mélanome cutané, d'un carcinome tel que cancer du côlon, cancer du poumon, adénocarcinome, cancer de la prostate, cancer de l'oesophage, cancer du sein, cancer du rein, d'un sarcome, d'un myélome, d'une leucémie, en particulier d'une LMA (leucémie myéloïde aiguë), d'un gliome (tumeur gliale), de lymphomes et de blastomes.

**Figur 1**

**Figur 2**

Figur 4

Figur 3

**Figur 5**

**Figur 6**

**Figur 7**

B

lacZ+ Zellen pro 20µm Schnitt

Relative vertikale Dimension [µm]

| lacZ (violett, dunkle Bereiche) | lacZ (violett + umrandet) | MHC II (orange, helle Bereiche) | lacZ (violett, + dunkle Ber.) | MHC II (grün, helle Bereiche) |

**Figur 8 A-E**

**Figur 9 A**

**Figur 9 B**

**Figur 10 (A, B, C, D)**

Figur 11

A  200μm  B

2,5mm

Innenseite

200μm

C

Knorpel

Innenseite

ventrale Epidermis

Aussenseite

dorsale Epidermis

**Figur 12 (A, B, C)**

Figur 13

lacZ (violett)  lacZ (violett umrandet)  lacZ (violett)
+ MHC II (orange)  + MHC II (grün)

**Figur 14**

lacZ + eGFP

blaugrün   rot   schwarz   rot   schwarz

100µm

X-gal   anti eGFP   X-gal + anti eGFP

blaugrün

**Figur 15**

**Figur 16**

EP 1 881 847 B2

Figur 17

42

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1083232 A
- WO 9914346 A
- US 5580859 A
- US 6214804 B
- WO 2005009346 A2
- US 2003143204 A1
- US 2007132788 A1
- US 2009106567 A1
- WO 2006008154 A
- US 4373071 A
- US 4401796 A
- US 4415732 A
- US 4458066 A
- US 4500707 A
- US 4668777 A
- US 4973679 A
- US 5047524 A
- US 5132418 A
- US 5153319 A
- US 5262530 A
- US 5700642 A

**In der Beschreibung aufgeführte Nicht-Patentliteratur**

- **CAPUT et al.** *Proc. Natl. Acad. Sci. USA,* 1986, vol. 83, 1670-1674
- **BINDER et al.** *EMBO J.,* 1994, vol. 13, 1969-1980
- **HOLCIK et al.** *Proc. Natl. Acad. Sci. USA,* 1997, vol. 94, 2410-2414
- **MANIATIS et al.** Molecular Cloning: A Laboratory Manual. Cold Spring Harbor Laboratory Press, 2001
- **ULMER J.B.** *Curr. Opin. DrugDiscov. Devel,* 2001, vol. 4, 192-197
- **J. A. WOLFF et al.** *Science,* 1990, vol. 247, 1465-1468
- **H. L. ROBINSON ; L. A. HUNT ; R. G. WEBSTER.** *Vaccine,* 1993, vol. 11, 957-960
- **J. B. ULMER et al.** *Science,* 1993, vol. 259, 1745-1749
- **D. BOCZKOWSKI ; S. K. NAIR ; D. SNYDER ; E. GILBOA.** *J.Exp.Med.,* 1996, vol. 184, 465-472
- **J. P. CARRALOT et al.** *Cell Mol.Life Sci.,* 2004
- **R. M. CONRY et al.** *Cancer Res.,* 1995, vol. 55, 1397-1400
- **R. D. GRANSTEIN ; W. DING ; H. OZAWA.** *J Invest Dermatol,* 2000, vol. 114, 632-636
- **I. HOERR ; R. OBST ; H. G. RAMMENSEE ; G. JUNG.** *Eur. J Immunol.,* 2000, vol. 30, 1-7
- **J. DONNELLY ; K. BERRY ; J. B. ULMER.** *Int J Parasitol.,* 2003, vol. 33, 457-467
- **D. M. KLINMAN et al.** *Springer Semin.Immunopatho,* 1997, vol. 19, 245-256
- **J.-P. CARRALOT et al.** *CMLS Cell. Mol. Life Sci.,* 2004, vol. 61, 2418-2424
- **P. FORG ; P. VON HOEGEN ; W. DALEMANS ; V. SCHIRRMACHER.** *Gene Ther.,* 1998, vol. 5, 789-797